# EUROPEAN PATENT SPECIFICATION

(11) **EP 0 543 924 B1**
(45) Date of publication and mention of the grant of the patent: **18.06.1997**
(21) Application number: 91916041.6
(22) Date of filing: 12.08.1991
(51) Int. Cl.: C12Q 1/70

(54) **NANBV DIAGNOSTICS: POLYNUCLEOTIDES USEFUL FOR SCREENING FOR HEPATITIS C VIRUS**
NANBV DIAGNOSTIK: FÜR DIE UNTERSUCHUNG AUF HEPATITIS C VERWENDBARE POLYNUKLEOTIDE
DIAGNOSTICS DU VIRUS NON A, NON B: POLYNUCLEOTIDES UTILES DANS LA DETECTION DU VIRUS DE L'HEPATITE C

(30) Priority: 10.08.1990 US 566209
(43) Date of publication of application: 02.06.1993
(73) Proprietor: CHIRON CORPORATION, Emeryville, California 94608 (US)
(72) Inventor: HOUGHTON, Michael, Danville, CA 94526 (US); CHOO, Qui-Lim, El Cerrito, CA 94530 (US); KUO, George, San Francisco, CA 94112 (US); WEINER, Amy, J., Benicia, CA 94510 (US); HAN, Jang, Lafayette, CA 94549 (US); URDEA, Michael, Steven, Alamo, CA 94501 (US); IRVINE, Bruce, Duncan, Concord, CA 94519 (US); KOLBERG, Janice, A., Hercules, CA 94547 (US)
(74) Representative: Goldin, Douglas Michael
(86) International application number: US9105728
(87) International publication number: WO9202642

(56) References cited:
- EP-A- 0 398 748
- EP-A- 0 461 863
- EP-A- 0 469 348
- WO-A-90/09457
- US-A- 4 683 195
- Journal of the Catholic Medical College, Vol. 43, No. 2, issued 1990, PARK et al., "Detection of Serum HBV DNA in Sporadic Non-A Non-B Hepatitis by using a Novel Double Polymerase Chain Reaction", pages 469-484, see the Abstract.

## Description

### Technical Field

The invention relates to materials and methodologies for managing the spread of non-A, non-B hepatitis virus (NANBV) infection. More specifically, it relates to an etiologic agent of non-A, non-B hepatitis (NANBH), hepatitis C virus (HCV), and to polynucleotides and analogs thereof, which are useful in assays for the detection of HCV in biological samples.

### References Cited in the Application

Barr et al. (1986), Biotechniques 4:428.
Beaucage et al. (1981), Tetrahedron Letters 22:1859.
Botstein (1979), Gene 8:17.
Brinton, M.A. (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press), p.327-374.
Broach (1981) in: Molecular Biology of the Yeast Saccharomyces, Vol. 1, p.445, Cold Spring Harbor Press.
Broach et al. (1983), Meth. Enz. 101:307.
Brown et al. (1979), Methods in Enzymology 68:109.
Byrne et al. (1988), Nucleic Acids Res. 16:4165.
Castle et al. (1986), Virology 119:10.
Chang et al. (1977), Nature 198:1056.
Chirgwin et al. (1979), Biochemistry 18:5294.
Choo et al. (1989), Science 244:359.
Chomczynski and Sacchi (1987), Analytical Biochemistry 162:156.
Clewell et al. (1969), Proc. Natl. Acad. Sci. USA 62:1159.
Clewell (1972), J. Bacteriol. 110:667.
Cohen (1972), Proc. Natl. Acad. Sci. USA 69:2110.
Cousens et al. (1987), Gene 61:265.
De Boer et al. (1983), Proc. Natl. Acad. Sci. USA 292:128.
Dreesman et al. (1985), J. Infect. Disease 151:761.
Feinstone et al. (1981), J. Inf. Dis. 144: 588.
Feinstone et al. (1983), Infection and Immunology 41:816.
Feinstone, S.M. and Hoofnagle, J.H. (1984), New Engl. J. Med. 311:185.
Fields & Knipe (1986), FUNDAMENTAL VIROLOGY (Raven Press, N.Y.).
Fiers et al. (1978), Nature 273:113.
Gerety, R.J. et al., in VIRAL HEPATITIS AND LIVER DISEASE (Vyas, B.N., Dienstag, J.L., and Hoofnagle, J.H., eds,
Grune and Stratton, Inc., 1984) pp 23-47.
Goeddel et al. (1980), Nucleic Acids Res. 8:4057.
Graham and Van der Eb (1978), Virology 52:546.
Grunstein and Hogness (1975), Proc. Natl. Acad. Sci. USA 73:3961.
Grych et al. (1985), Nature 316:74.
Gubler and Hoffman (1983), Gene 25:263.
Hahn et al. (1988), Virology 162:167.
Han (1987), Biochemistry 26:1617.
Hammerling et al. (1981), MONOCLONAL ANTIBODIES AND T-CELL HYBRIDOMAS.
Hess et al. (1968), J. Adv. Enzyme Reg 7:149.
Hinnen et al. (1978), Proc. Natl. Acad. Sci. 75:1929.
Hitzeman et al. (1980), J. Biol. Chem. 255:2073.
Holland et al. (1978), Biochemistry 17:4900.
Holland (1981), J. Biol. Chem. 256: 1385.
Houghton et al. (1981), Nucleic Acids Res. 9:247
Huynh, T.V. et al. (1985) in DNA CLONING TECHNIQUES; A PRACTICAL APPROACH (D. Glover, Ed., IRL Press, Oxford, U.K.) pp. 49-78.
Immun. Rev. (1982) 62:185.
Iwarson (1987), British Medical J. 295:946.
Kennett et al. (1980) MONOCLONAL ANTIBODIES.
Kuo et al. (1989), Science 244:362.
Kyte and Doolittle (1982), J. Mol. Biol. 157:105-132.
Landegren et al. (1988), Science 242:229.
Maniatis, T., et al. (1982) MOLECULAR CLONING; A LABORATORY MANUAL (Cold Spring Harbor Press, Cold Spring Harbor, N.Y.).
Matthews and Kricka (1988), Analytical Biochemistry 169:1.
METHODS IN ENZYMOLOGY (Academic Press).
Mittlin (1989), Clinical Chem. 35:1819.
Laemmli (1970), Nature 227, 680.
Lee et al. (1988), Science 239:1288.
Loh et al. (1989), Science 243:217.
Mackow et al. (1987), Virology 159:217.
Mayer and Walker, eds. (1987), IMMUNOCHEMICAL METHODS IN CELL AND MOLECULAR BIOLOGY (Academic Press, London).
Mayumi et al. (1990), Japanese J. Exp. Med. 60:167.
Maxam et al. (1980), Methods in Enzymology 65:499.
MacNamara et al. (1984), Science 226:1325.
Messing et al. (1981), Nucleic Acids Res. 9:309.
Messing (1983), Methods in Enzymology 101:20-37. METHODS IN ENZYMOLOGY (Academic Press).
Michelle et al., Int. Symposium on Viral Hepatitis.
Monath (1986) in THE VIRUSES: THE TOGAVIRADAE AND FLAVIVIRIDAE (Series eds. Fraenkel-Conrat and Wagner, vol. eds. Schlesinger and Schlesinger, Plenum Press), p.375-440.
Murakawa et al. (1988), DNA 7:287.
Nagahuma et al. (1984), Anal. Biochem. 141:74.
Narang et al (1979), Methods in Enzymology 68:90.
Neurath et al. (1984), Science 224:392.
Nisonoff et al. (1981), Clin. Immunol. Immunopathol. 21:397-406.
Overby, L.R. (1985), Curr. Hepatol. 5:49.
Overby, L.R. (1986), Curr. Hepatol. 6:65.
Overby, L.R. (1987), Curr. Hepatol. 7:35.
Peleg (1969), Nature 221:193.
Pfefferkorn and Shapiro (1974), in COMPREHENSIVE VIROLOGY, Vol. 2 (Fraenkel-Conrat & Wagner, eds., Plenum, N.Y.) pp. 171-230.
Prince, A.M. (1983), Annu. Rev. Microbiol. 37:217.
Rice et al. (1985), Science 229:726.
Rice et al. (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press), p.279-328.
Roehrig (1986) in THE VIRUSES: THE TOGAVIRIDAE AND FLAVIVIRIDAE (Series eds. Fraenkel-Conrat and Wagner, vol. eds. Schlesinger and Schlesinger, Plenum Press)
Rosenberg et al. (1984), Nature 312:7.
Sadler et al. (1980), Gene 8, 279.
Saiki et al. (1985), Science 230:1350.
Saiki et al. (1986), Nature 324: 163.
Saiki et al. (1988), Science 239:487.
Sanger et al. (1977), Proc. Natl. Acad. Sci. USA 74:5463.
Scharf et al. (1986), Science 233:1076.
Schlesinger et al. (1986), J. Virol. 60:1153.
Schreier, M., et al. (1980) HYBRIDOMA TECHNIQUES
Scopes (1984), PROTEIN PURIFICATION, PRINCIPLES AND PRACTICE, SECOND EDITION (Springer-Verlag, N.Y.).
Shimatake et al. (1981), Nature 292:128.
Shigekawa and Dower (1988), BioTechniques 6:742.
Steimer et al. (1986), J. Virol. 58:9.
Stollar (1980), in THE TOGAVIRUSES (R.W. Schlesinger, ed., Academic Press, N.Y.), pp. 584-622.
Sumiyoshi et al. (1987), Virology 161:497.
Taylor et al. (1976), Biochem. Biophys. Acta 442:324.
Towbin et al. (1979), Proc. Natl. Acad. Sci. USA 76, 4350.
Tsu and Herzenberg (1980), in SELECTED METHODS IN CELLULAR IMMUNOLOGY (W.H. Freeman and Co.) pp. 373-391.
Vytdehaag et al. (1985), J. Immunol. 134:1225.
Valenzuela, P., et al. (1982), Nature 298:344.
Valenzuela, P., et al. (1984), in HEPATITIS B (Millman, I., et al., ed, Plenum Press) pp. 225-236.
Warner (1984), DNA 3:401.
Wu and Grossman (1987), Methods in Enzymology Vol. 154, RECOMBINANT DNA, Part E.
Wu (1987), Methods in Enzymology vol 155, RECOMBINANT DNA, part F.
Zoller (1982), Nucleic Acids Res. 10:6487.

### Cited Patents

U.S. Patent No. 4,341,761
U.S. Patent No. 4,399,121
U.S. Patent No. 4,427,783
U.S. Patent No. 4,444,887
U.S. Patent No. 4,466,917
U.S. Patent No. 4,472,500
U.S. Patent No. 4,491,632
U.S. Patent No. 4,493,890
U.S. Patent No. 4,683,202
U.S. Patent No. 4,458,066
U.S. Patent No. 4,868,105

### Background Art

Non-A, Non-B hepatitis (NANBH) is a transmissible disease or family of diseases that are believed to be viral-induced, and that are distinguishable from other forms of viral-associated liver diseases, including that caused by the known hepatitis viruses, i.e., hepatitis A virus (HAV), hepatitis B virus (HBV), and delta hepatitis virus (HDV), as well as the hepatitis induced by cytomegalovirus (CMV) or Epstein-Barr virus (EBV). NANBH was first identified in transfused individuals. Transmission from man to chimpanzee and serial passage in chimpanzees provided evidence that NANBH is due to a transmissible infectious agent or agents.

Epidemiologic evidence is suggestive that there may be three types of NANBH: the water-borne epidemic type; the blood or needle associated type; and the sporadically occurring (community acquired) type. However, the number of agents which may be the causative of NANBH are unknown.

There have been a number of candidate NANBV. See, for example the reviews by Prince (1983), Feinstone and Hoofnagle (1984), and Overby (1985, 1986, 1987) and the article by Iwarson (1987). However, there is no proof that any of these candidates represent the etiological agent of NANBH.

The demand for sensitive, specific methods for screening and identifying carriers of NANBV and NANBV contaminated blood or blood products is significant. Post-transfusion hepatitis (PTH) occurs in approximately 10% of transfused patients, and NANBH accounts for up to 90% of these cases. The major problem in this disease is the frequent progression to chronic liver damage (25-55%).

Patient care as well as the prevention of transmission of NANBH by blood and blood products or by close personal contact require reliable screening, diagnostic and prognostic tools to detect nucleic acids, antigens and antibodies related to NANBV.

Methods for detecting specific polynucleotides by hybridization assays are known in the art. See, for example, Matthews and Kricka (1988), Analytical Biochemistry 169:1; Landegren et al. (1988), Science 242:229; and Mittlin (1989), Clinical chem. 35:1819. U.S. Patent No. 4,868,105, issued Sept. 9, 1989, and in EPO Pub. No. 225807 (published June 16, 1987).

### Disclosure of the Invention

Methods for isolating and/or detecting specific polynucleotides by hybridization could not be used for screening for HCV until Applicants' discovery of HCV. The Applicants' invention provides materials and methods for obtaining the viral genomic sequences, which are provided in PCT Pub. No. WO90/14436, and infra.

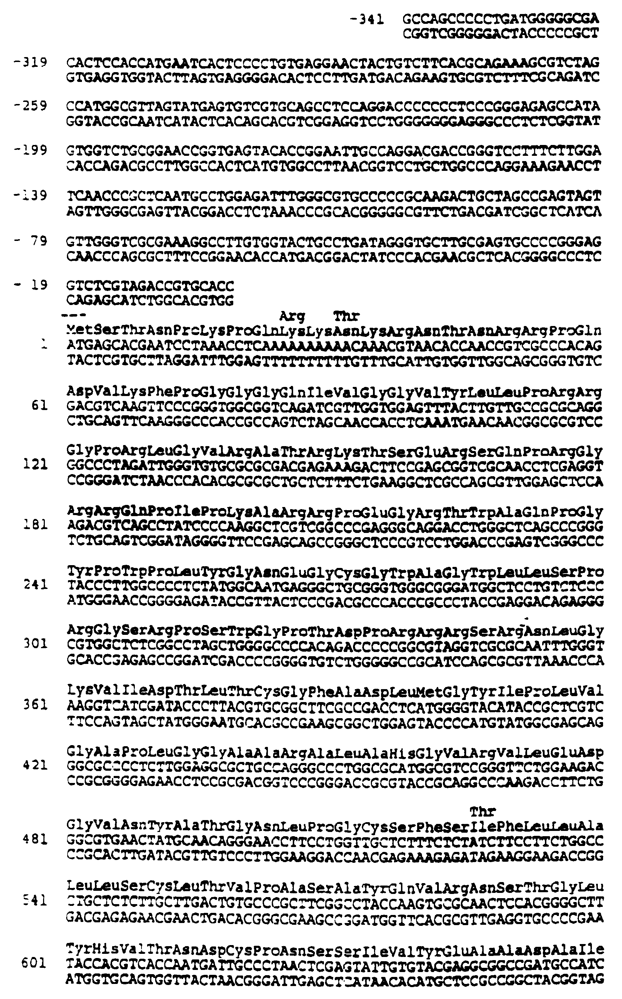

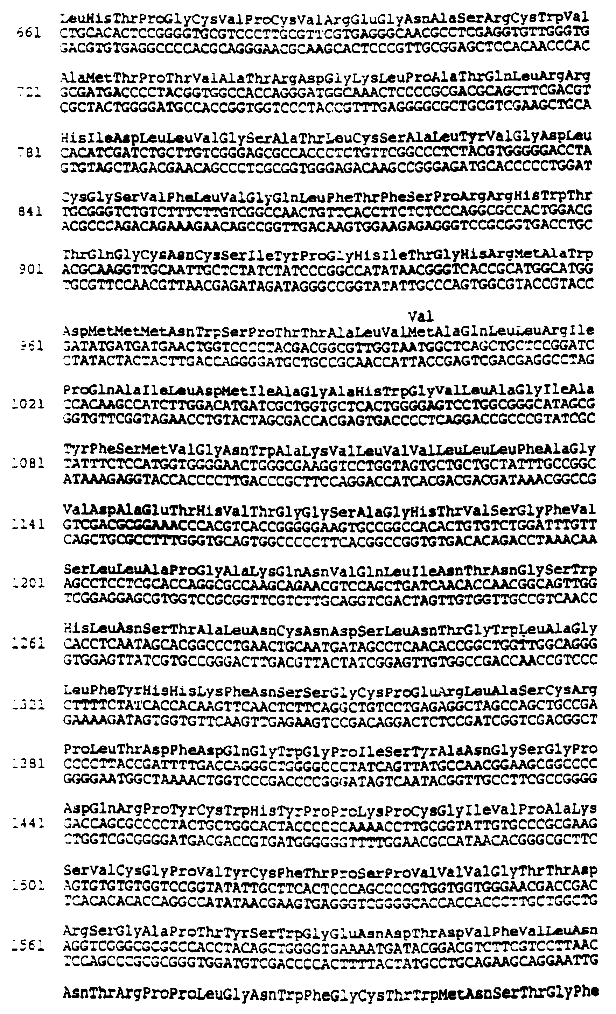

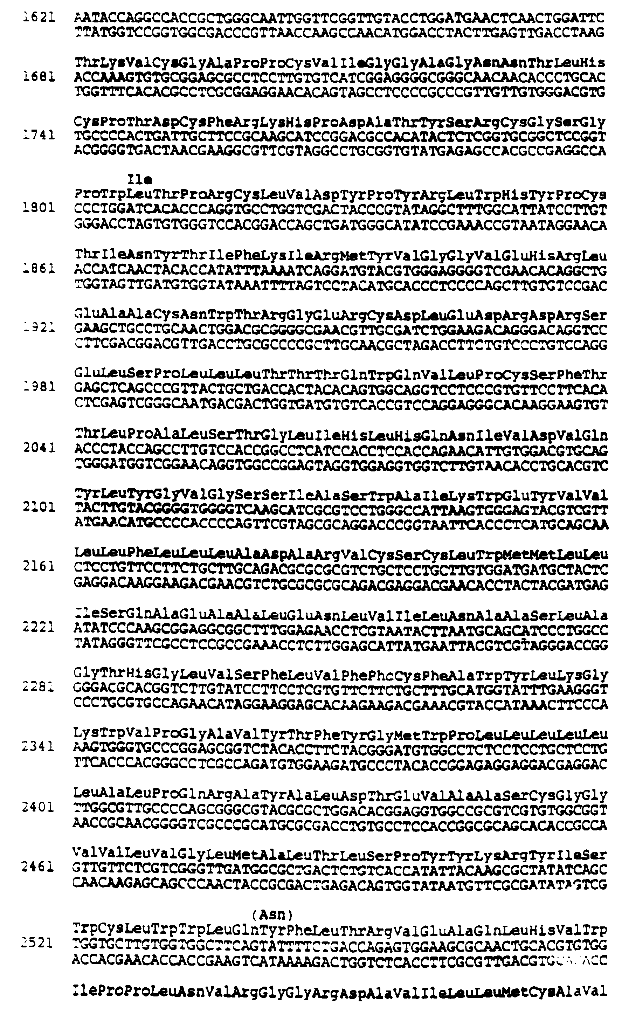

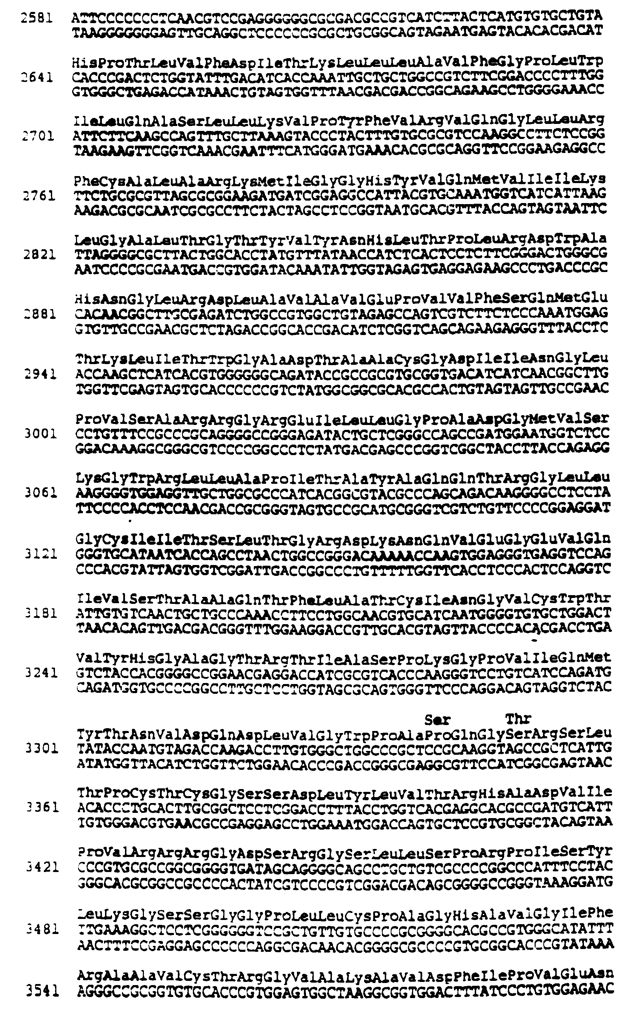

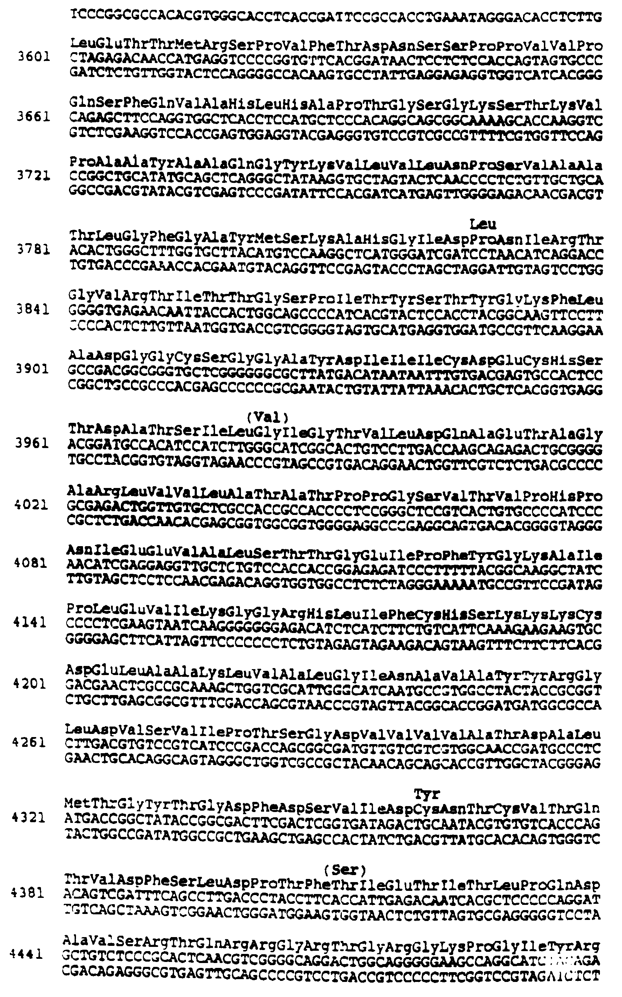

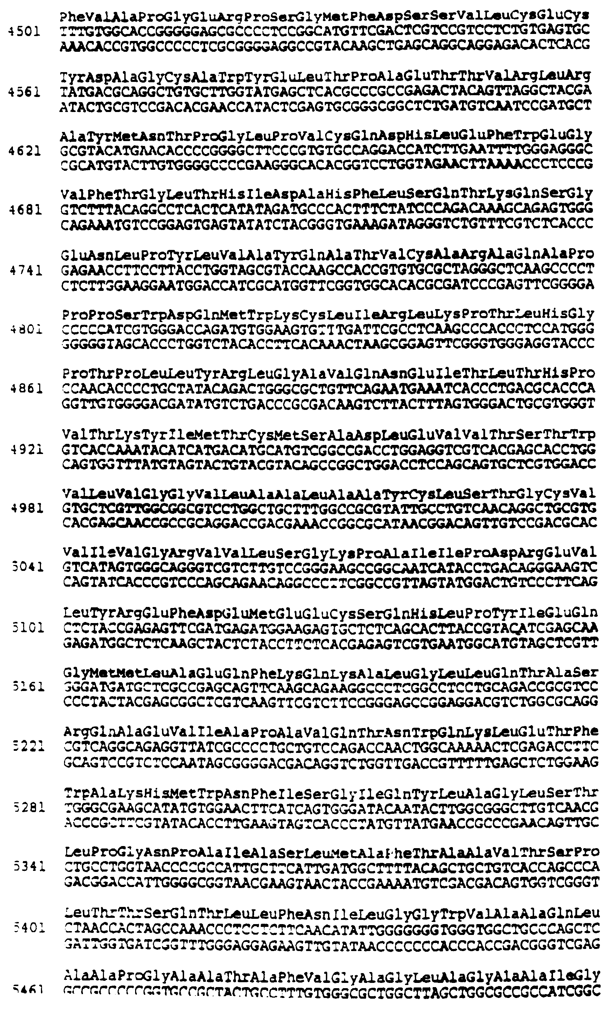

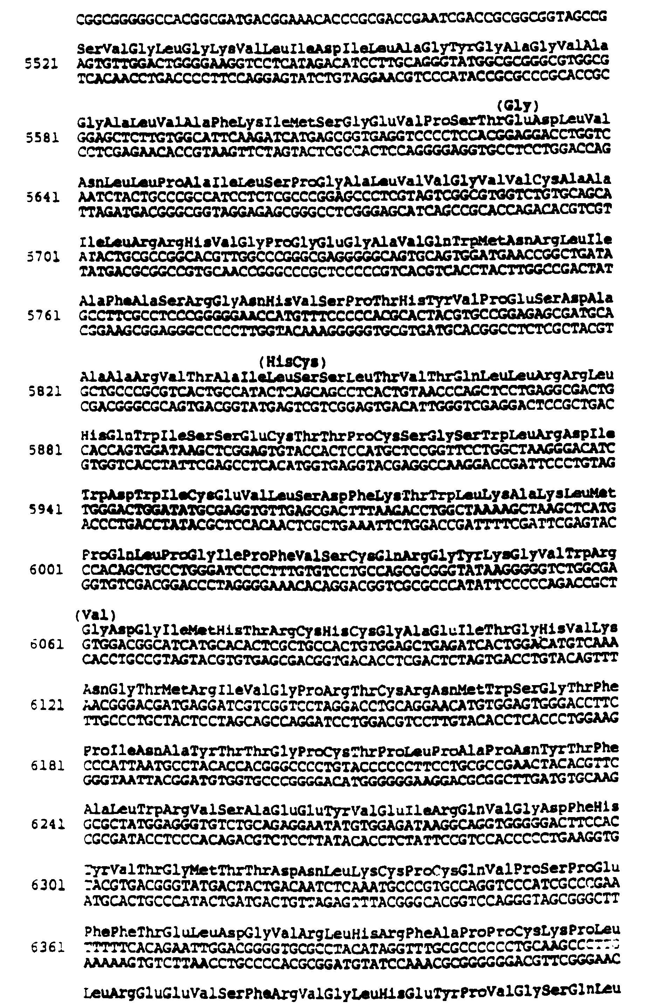

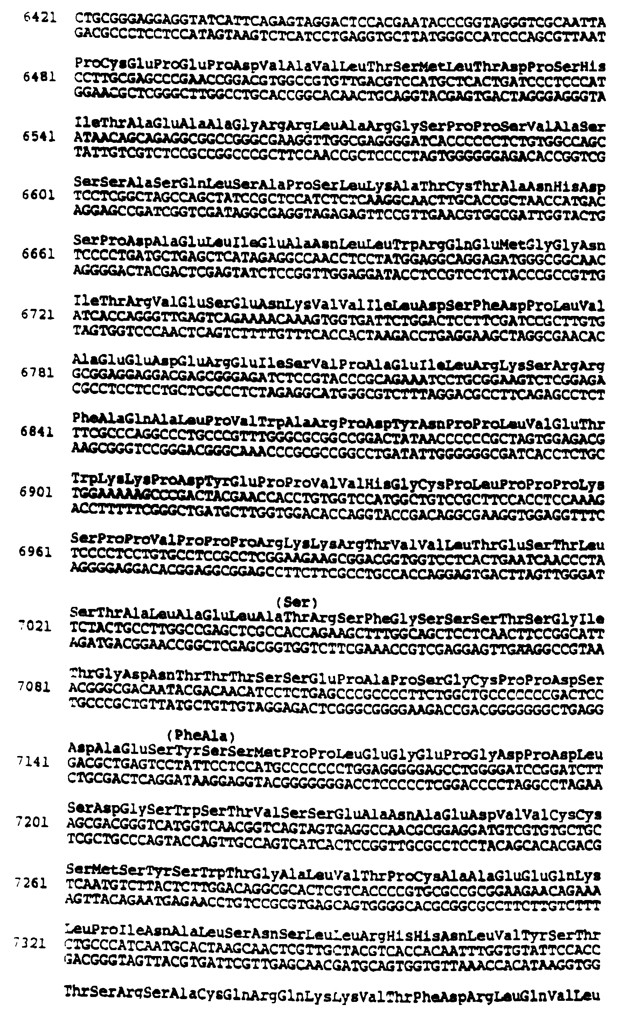

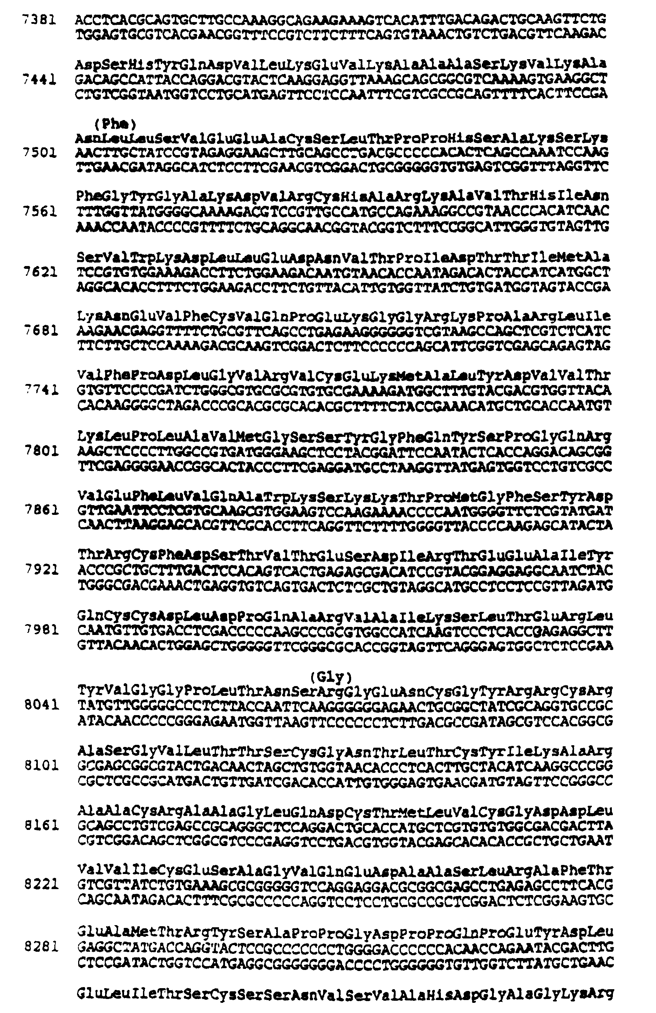

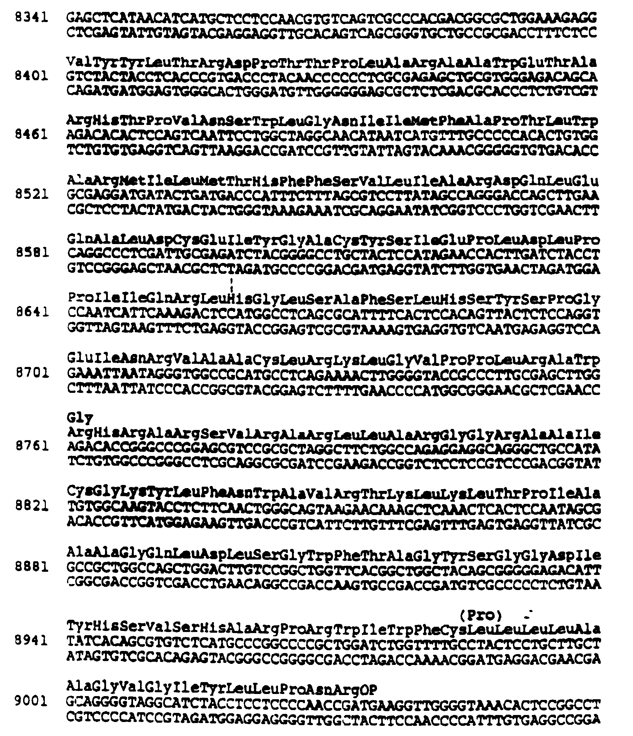

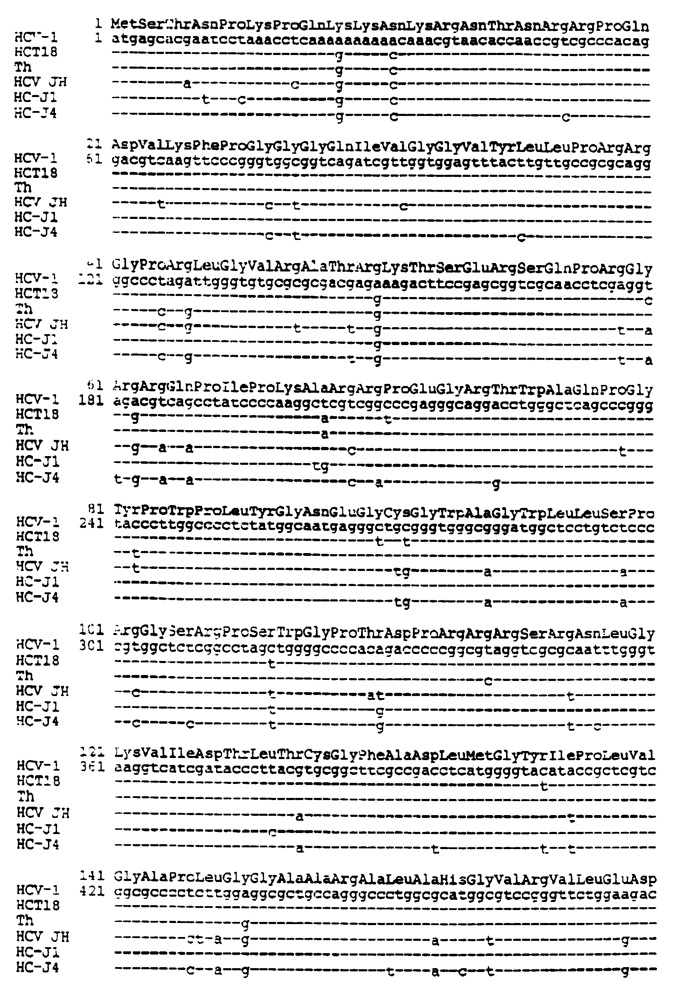

The present invention thus provides a process for detecting an HCV sequence in an analyte strand suspected of containing an HCV polynucleotide, wherein the HCV polynucleotide comprises a selected target region, said process comprising:
(a) providing an oligonucleotide capable of hybridizing to an HCV sequence in an analyte polynucleotide strand, wherein the oligonucleotide consists essentially of (i): a polynucleotide sequence selected from the group consisting of oligonucleotides at least 12 nucleotides in length complementary to the following regions of the HCV genome (as shown in Figure 1): 16-45, 49-78, 82-111, 115-144, 148-177, 211-240, 242-271, 275-304, 332-361, 365-394, 398-427, and 457-486, optionally linked to (ii) a polynucleotide other than that to which it is linked in nature;
(b) incubating the analyte strand with the oligonucleotide of (a) which allow specific hybrid duplexes to form between the targeting sequence and the target sequence; and
(c) detecting hybrids formed between target region, if any, and the oligonucleotide.

The present invention also provides a process for detecting the presence or absence of a single or double-stranded specific nucleic acid sequence in a sample containing a nucleic acid or mixture thereof, which process comprises:
(a) treating the sample with one oligonucleotide primer for each strand of the specific nucleotide sequence, allowing specific hybrid duplexes to form between the oligonucleotide primer and the specific sequence;
(b) synthesizing a primer extension product; and
(c) detecting hybrids formed between the specific nucleotide sequence, if any, and the oligonucleotide primer; characterized in that the oligonucleotide primers of (a) are as defined above or the complement thereof.

Step (a) above may be repeated, either with the same oligonucleotide or with another oligomer(s), wherein the other oligomer(s) are as defined above.

Yet another aspect of the invention is a method for eliminating blood contaminated with an infectious agent from a blood supply made up of units from individual blood donors comprising:
(a) providing analyte nucleic acids from a sample of blood suspected of containing a target sequence of a viral agent;
(b) providing an oligomer capable of hybridizing to the target sequence, if present, in the analyte nucleic acids, characterized in that
   (i) the infectious agent is HCV; and
   (ii) the oligomer consists essentilly of (i): a polynucleotide sequence selected from the group consisting of oligonucleotides at least 12 nucleotides in length complementary to the following regions of the HCV genome (as shown in Figure 1): 16-45, 49-78, 82-111, 115-144, 148-177, 211-240, 242-271, 275-304, 332-361, 365-394, 398-427, and 457-486, optionally linked to (ii) a polynucleotide other than that to which it is linked in nature;
(c) reacting (a) and (b) under conditions which allow the formation of a polynucleotide duplex between the targeting sequence and the target sequence, if any;
(d) detecting a duplex formed in (c), if any; and
(e) saving the blood from which complexes were not detected in (d).

The invention further provides a reagent useful for detecting of HCV, wherein said reagent comprises an oligonucleotide consisting essentially of polynucleotide sequence selected from the group consisting of oligonucleotides at least 12 nucleotides in length complementary to the following regions of the HCV genome (as shown in Figure 1): 16-45, 49-78, 82-111, 115-144, 148-177, 211-240, 242-271, 275-304, 332-361, 365-394, 398-427, and 457-486, optionally linked to (ii) a polynucleotide other than that to which it is linked in nature.

### Brief Description of the Drawings

Fig. 1 shows the compiled HCV cDNA sequence derived from the clone described herein and from the compiled HCV cDNA sequence presented in PCT Pub. No. WO90/14436. The clones from which the sequence was derived are 5'-clone32, b114a, 18g, ag30a, CA205a, CA290a, CA216a, pi14a, CA167b, CA156e, CA84a, CA59a, K9-1 (also called k9-1), 26j, 13i, 12f, 14i, 11b, 7f, 7e, 8h, 33c, 40b, 37b, 35, 36, 81, 32, 33b, 25c, 14c, 8f, 33f, 33g, 39c, 35f, 19g, 26g, 15e, b5a, 16jh, 6k, and pl31jh. In the figure the three horizontal dashes above the sequence indicate the position of the putative initiator methionine codon. Also shown in the figure is the amino acid sequence of the putative polyprotein encoded in the HCV cDNA. Heterogeneities in cloned DNAs of HCV1 are indicated by the amino acids indicated above the putatively encoded sequence of the large ORF; the parentheses indicate that the heterogeneity was detected at or near to the 5'- or 3'- end of the HCV cDNA in the clone.

Fig. 2 shows the DNA consensus sequences for five different HCV isolates from different geographic locations (Japan and US), where the amino acids encoded by the large ORF of HCV1 are shown above the DNA sequences.

Fig. 3 shows the sequences of labelling probes for the detection of HCV RNA in biological samples used in Example III.

Fig. 4 shows the alignment of the probes in Fig. 3 with HCV1, according to the numbering in Fig. 1.

### Modes for Carrying Out the Invention

The term "hepatitis C virus" (HCV) has been reserved by workers in the field for an heretofore unknown etiologic agent of NANBH. The prototype isolate of HCV has been identified in EP-A-318 216. The term HCV also includes new isolates of the same viral species. As an extension of this terminology, the disease caused by HCV, formerly called blood-borne NANB hepatitis (BB-NANBH), is called hepatitis C. The terms NANBH and hepatitis C may be used interchangeably herein.

HCV is a viral species of which pathogenic strains cause BB-NANBH. There may also be attenuated strains or defective interfering particles derived therefrom. As shown infra, the HCV genome is comprised of RNA. It is known that RNA containing viruses have relatively high rates of spontaneous mutation, i.e., reportedly on the order of 10⁻³ to 10⁻⁴ per incorporated nucleotide (Fields & Knipe (1986)). Therefore, since heterogeneity and fluidity of genotype are inherent in RNA viruses, there are multiple strains/isolates, which may be virulent or avirulent, within the HCV species. The compositions and methods described herein, enable the propagation, identification, detection, and isolation of the various HCV strains or isolates.

Several different strains/isolates of HCV have been identified (See PCT Pub. No. WO90/14436). One such strain or isolate, which is a prototype, is named CDC/HCV1 (also called HCV1). Information from one strain or isolate, such as a partial genomic sequence, is sufficient to allow those skilled in the art using standard techniques to isolate new strains/isolates and to identify whether such new strains/isolates are HCV. For example, several different strains/isolates are described infra. These strains, which were obtained from a number of human sera (and from different geographical areas), were isolated utilizing the information from the genomic sequence of HCV1.

Using the techniques described in PCT Pub. No. WO90/14436, the genomic structure and the nucleotide sequence of HCV1 genomic RNA has been deduced. The genome appears to be single-stranded RNA containing ∼ 10,000 nucleotides. The genome is positive-stranded, and possesses a continuous, translational open reading frame (ORF) that encodes a polyprotein of about 3,000 amino acids. In the ORF, the structural protein(s) appear to be encoded in approximately the first quarter of the N-terminus region, with the majority of the polyprotein responsible for non-structural proteins. When compared with all known viral sequences, small but significant co-linear homologies are observed with the non-structural proteins of the flavivirus family, and with the pestiviruses (which are now also considered to be part of the Flavivirus family).

The flavivirus polyprotein contains, from the amino terminus to the carboxy terminus, the nucleocapsid protein (C), the matrix protein (M), the envelope protein (E), and the non-structural proteins (NS) 1, 2(a+b), 3, 4(a+b), and 5. Based upon the putative amino acids encoded in the nucleotide sequence of HCV1, a small domain at the extreme N-terminus of the HCV polyprotein appears similar both in size and high content of basic residues to the nucleocapsid protein (C) found at the N-terminus of flaviviral polyproteins. The non-structural proteins 2,3,4, and 5 (NS2-5) of HCV and of yellow fever virus (YFV) appear to have counterparts of similar size and hydropathicity, although there is divergence of the amino acid sequences. However, the region of HCV which would correspond to the regions of YFV polyprotein which contains the M, E, and NS1 protein not only differs in sequence, but also appears to be quite different both in size and hydropathicity. Thus, while certain domains of the HCV genome may be referred to herein as, for example, NS1, or NS2, it should be borne in mind that these designations are speculative; there may be considerable differences between the HCV family and flaviviruses that have yet to be appreciated.

Different strains, isolates or subtypes of HCV are expected to contain variations at the amino acid and nucleic acids compared with HCV1. Many isolates are expected to show much (i.e., more than about 40%) homology in the total amino acid sequence compared with HCV1. However, it may also be found that there are other less homologous HCV isolates. These would be defined as HCV according to various criteria such as, for example, an ORF of approximately 9,000 nucleotides to approximately 12,000 nucleotides, encoding a polyprotein similar in size to that of HCV1, an encoded polyprotein of similar hydrophobic and/or antigenic character to that of HCV1, and the presence of co-linear peptide sequences that are conserved with HCV1. In addition, it is believed that the genome would be a positive-stranded RNA.

All HCV isolates encode at least one epitope which is immunologically identifiable (i.e., immunologically cross-reactive) with an epitope encoded in the HCV cDNAs described herein. Preferably the epitope is contained in an amino acid sequence described herein and is unique to HCV when compared to previously known pathogens. The uniqueness of the epitope may be determined by its immunological reactivity with anti-HCV antibodies and lack of immunological reactivity with antibodies to known pathogens.

HCV strains and isolates are evolutionarily related. Therefore, it is expected that the overall homology of the genomes at the nucleotide level may be about 40% or greater, probably will be about 50% or greater, probably about 60% or greater, and even more probably about 80% or greater; and in addition that there will be corresponding contiguous sequences of at least about 13 nucleotides. It should be noted that there are variable and hypervariable regions within the HCV genome; therefore, the homology in these regions is expected to be significantly less than that in the overall genome. The correspondence between the putative HCV strain genomic sequence and, for example, the CDC/ HCV1 cDNA sequence can be determined by techniques known in the art. For example, they can be determined by a direct comparison of the sequence information of the polynucleotide from the putative HCV, and the HCV cDNA sequence(s) described herein. They also can be determined by hybridization of the polynucleotides under conditions which form stable duplexes between homologous regions (for example, those which would be used prior to S₁ digestion), followed by digestion with single stranded specific nuclease(s), followed by size determination of the digested fragments.

Because of the evolutionary relationship of the strains or isolates of HCV, putative HCV strains or isolates are identifiable by their homology at the polypeptide level. Generally, HCV strains or isolates are expected to be at least 40% homologous, more than about 50% homologous, probably more than about 70% homologous, and even more probably more than about 80% homologous, and some may even be more than about 90% homologous at the polypeptide level. The techniques for determining amino acid sequence homology are known in the art. For example, the amino acid sequence may be determined directly and compared to the sequences provided herein. Alternatively the nucleotide sequence of the genomic material of the putative HCV may be determined (usually via a cDNA intermediate), the putative amino acid sequence encoded therein can be determined, and the corresponding regions compared.

As used herein, a polynucleotide "derived from" a designated sequence refers to a polynucleotide sequence which is comprised of a sequence of approximately at least about 6 nucleotides, preferably at least about 8 nucleotides, more preferably at least about 10-12 nucleotides, and even more preferably at least about 15-20 nucleotides corresponding to a region of the designated nucleotide sequence. "Corresponding" means homologous to or complementary to the designated sequence. Preferably, the sequence of the region from which the polynucleotide is derived is homologous to or complementary to a sequence which is unique to an HCV genome. More preferably, the derived sequence is homologous or complementary to a sequence that is unique to all or to a majority of HCV isolates. Whether or not a sequence is unique to the HCV genome can be determined by techniques known to those of skill in the art. For example, the sequence can be compared to sequences in databanks, e.g., Genebank, to determine whether it is present in the uninfected host or other organisms. The sequence can also be compared to the known sequences of other viral agents, including those which are known to induce hepatitis, e.g., HAV, HBV, and HDV, and to members of the Flaviviridae. The correspondence or non-correspondence of the derived sequence to other sequences can also be determined by hybridization under the appropriate stringency conditions. Hybridization techniques for determining the complementarity of nucleic acid sequences are known in the art, and are discussed infra. See also, for example, Maniatis et al. (1982). In addition, mismatches of duplex polynucleotides formed by hybridization can be determined by known techniques, including for example, digestion with a nuclease such as S1 that specifically digests single-stranded areas in duplex polynucleotides. Regions from which typical DNA sequences may be "derived" include but are not limited to, for example, regions encoding specific epitopes, as well as non-transcribed and/or non-translated regions.

The derived polynucleotide is not necessarily physically derived from the nucleotide sequence shown, but may be generated in any manner, including for example, chemical synthesis or DNA replication or reverse transcription or transcription. In addition, combinations of regions corresponding to that of the designated sequence may be modified in ways known in the art to be consistent with an intended use.

The term "recombinant polynucleotide" as used herein intends a polynucleotide of genomic, cDNA, semisynthetic, or synthetic origin which, by virtue of its origin or manipulation: (1) is not associated with all or a portion of a polynucleotide with which it is associated in nature, (2) is linked to a polynucleotide other than that to which it is linked in nature, or (3) does not occur in nature.

The term "polynucleotide" as used herein refers to a polymeric form of nucleotides of any length, either ribonucleotides or deoxyribonucleotides. This term refers only to the primary structure of the molecule. Thus, this term includes double- and single-stranded DNA and RNA. It also includes known types of modifications, for example, labels which are known in the art, methylation, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example proteins (including, e.g., nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide.

As used herein, the "sense strand" of a nucleic acid contains the sequence that has sequence homology to that of mRNA. The "anti-sense strand" contains a sequence which is complementary to that of the "sense strand".

As used herein, a "positive stranded genome" of a virus is a genomic polynucleotide, whether RNA or DNA, which encodes at least one viral polypeptide. Examples of positive stranded RNA viruses include Togaviridae, Coronaviridae, Retroviridae, Picornaviridae, and Caliciviridae. Included also, are the Flaviviridae, which were formerly classified as Togaviradae. These viruses are typically single stranded. See Fields & Knipe (1986).

The term "primer" as used herein refers to an oligomer which is capable of acting as a point of initiation of synthesis of a polynucleotide strand when placed under appropriate conditions. The primer will be completely or substantially complementary to a region of the polynucleotide strand to be copied. Thus, under conditions conducive to hybridization, the primer will anneal to the complementary region of the analyte strand. Upon addition of suitable reactants, (e.g., a polymerase, nucleotide triphosphates, and the like), the primer is extended by the polymerizing agent to form a copy of the analyte strand. The primer may be single-stranded, or alternatively may be partially or fully double-stranded.

The terms "analyte polynucleotide" and "analyte strand" refer to a single- or double-stranded nucleic acid molecule which is suspected of containing a target sequence, and which may be present in a biological sample.

As used herein, the term "oligomer" refers to primers and to probes. The term oligomer does not connote the size of the molecule. However, typically oligomers are no greater than 1000 nucleotides, more typically are no greater than 500 nucleotides, even more typically are no greater than 250 nucleotides; they may be no greater than 100 nucleotides, and may be no greater than 75 nucleotides, and also may be no greater than 50 nucleotides in length.

As used herein, the term "probe" refers to a structure comprising a polynucleotide which forms a hybrid structure with a target sequence, due to complementarity of at least one sequence in the probe with a sequence in the target region. The polynucleotide regions of probes may be composed of DNA, and/or RNA, and/or synthetic nucleotide analogs. Included within probes are "capture probes" and "label probes". Preferably the probe does not contain a sequence complementary to sequence(s) used to prime the polymerase chain reaction (PCR).

As used herein, the term "target region" refers to a region of the nucleic acid which is to be amplified and/or detected. The term "target sequence" refers to a sequence with which a probe or primer will form a stable hybrid under desired conditions.

The term "capture probe" as used herein refers to a polynucleotide comprised of a single-stranded polynucleotide coupled to a binding partner. The single-stranded polynucleotide comprises a targeting polynucleotide sequence, which is complementary to a target sequence in a target region to be detected in the analyte polynucleotide. This complementary region is of sufficient length and complementarity to the target sequence to afford a duplex of stability which is sufficient to immobilize the analyte polynucleotide to a solid surface (via the binding partners). The binding partner is specific for a second binding partner; the second binding partner can be bound to the surface of a solid support, or may be linked indirectly via other structures or binding partners to a solid support.

The term "targeting polynucleotide sequence" as used herein, refers to a polynucleotide sequence which comprises nucleotides which are complementary to a target nucleotide sequence; the sequence is of sufficient length and complementarity with the target sequence to form a duplex which has sufficient stability for the purpose intended.

The term "binding partner" as used herein refers to a molecule capable of binding a ligand molecule with high specificity, as for example an antigen and an antibody specific therefor. In general, the specific binding partners must bind with sufficient affinity to immobilize the analyte copy/complementary strand duplex (in the case of capture probes) under the isolation conditions. Specific binding partners are known in the art, and include, for example, biotin and avidin or streptavidin, IgG and protein A, the numerous known receptor-ligand couples, and complementary polynucleotide strands. In the case of complementary polynucleotide binding partners, the partners are normally at least about 15 bases in length, and may be at least 40 bases in length; in addition, they have a content of Gs and Cs of at least about 40% and as much as about 60%. The polynucleotides may be composed of DNA, RNA, or synthetic nucleotide analogs.

The term "coupled" as used herein refers to attachment by covalent bonds or by strong non-covalent interactions (e.g., hydrophobic interactions, hydrogen bonds, etc.). Covalent bonds may be, for example, ester, ether, phosphoester, amide, peptide, imide, carbon-sulfur bonds, carbon-phosphorus bonds, and the like.

The term "support" refers to any solid or semi-solid surface to which a desired binding partner may be anchored. Suitable supports include glass, plastic, metal, polymer gels, and the like, and may take the form of beads, wells, dipsticks, membranes, and the like.

The term "label" as used herein refers to any atom or moiety which can be used to provide a detectable (preferably quantifiable) signal, and which can be attached to a polynucleotide or polypeptide.

As used herein, the term "label probe" refers to an oligomer which comprises targeting polynucleotide sequence, which is complementary to a target sequence to be detected in the analyte polynucleotide. This complementary region is of sufficient length and complementarity to the target sequence to afford a duplex comprised of the "label probe" and the "target sequence" to be detected by the label. The oligomer is coupled to a label either directly, or indirectly via a set of ligand molecules with high specificity for each other. Sets of ligand molecules with high specificity are described supra, and also includes multimers.

The term "multimer", as used herein, refers to linear or branched polymers of the same repeating single-stranded polynucleotide unit or different single-stranded polynucleotide units. At least one of the units has a sequence, length, and composition that permits it to hybridize specifically to a first single-stranded nucleotide sequence of interest, typically an analyte or an oligomer (e.g., a label probe) bound to an analyte. In order to achieve such specificity and stability, this unit will normally be at least about 15 nucleotides in length, typically no more than about 50 nucleotides in length, and preferably about 30 nucleotides in length; moreover, the content of Gs and Cs will normally be at least about 40%, and at most about 60%. In addition to such unit(s), the multimer includes a multiplicity of units that are capable of hybridizing specifically and stably to a second single-stranded nucleotide of interest, typically a labeled polynucleotide or another multimer. These units are generally about the same size and composition as the multimers discussed above. When a multimer is designed to be hybridized to another multimer, the first and second oligonucleotide units are heterogeneous (different), and do not hybridize with each other under the conditions of the selected assay. Thus, multimers may be label probes, or may be ligands which couple the label to the probe.

As used herein, the term "viral RNA", which includes HCV RNA, refers to RNA from the viral genome, fragments thereof, transcripts thereof, and mutant sequences derived therefrom.

As used herein, a "biological sample" refers to a sample of tissue or fluid isolated from an individual, including but not limited to, for example, plasma, serum, spinal fluid, lymph fluid, the external sections of the skin, respiratory, intestinal, and genitourinary tracts, tears, saliva, milk, blood cells, tumors, organs, and also samples of in vitro cell culture constituents (including but not limited to conditioned medium resulting from the growth of cells in cell culture medium, putatively virally infected cells, recombinant cells, and cell components).

### Description of the Invention

The practice of the present invention will employ, unless otherwise indicated, conventional techniques of chemistry, molecular biology, microbiology, recombinant DNA, and immunology, which are within the skill of the art. Such techniques are explained fully in the literature. See e.g., Maniatis, Fitsch & Sambrook, "Molecular Cloning; A Laboratory Manual (1982); DNA Cloning, Volumes I and II (D.N Glover ed. 1985); Oligonucleotide Synthesis (M.J. Gait ed, 1984); Nucleic Acid Hybridization (B.D. Hames & SJ. Higgins eds. 1984); the series, Methods in Enzymology (Academic Press, Inc.), particularly Vol. 154 and Vol. 155 (Wu and Grossman, eds.).

The useful materials and processes of the present invention are made possible by the identification of HCV as the etiologic agent of BB-NANBV, and by the provision of a family of nucleotide sequences isolated from cDNA libraries which contain HCV cDNA sequences. These cDNA libraries were derived from nucleic acid sequences present in the plasma of an HCV-infected chimpanzee. The construction of one of these libraries, the "c" library (ATCC No. 40394), is described in PCT Pub. No. WO90/14436.

Utilizing the above-described HCV cDNA sequences, as well as that described herein, oligomers can be constructed which are useful as reagents for detecting viral polynucleotides in biological samples. For example, from the sequences it is possible to synthesize DNA oligomers of 12 nucleotides, or larger, which are useful as hybridization probes to detect the presence of HCV RNA in, for example, donated blood, blood fractions, sera of subjects suspected of harboring the virus, or cell culture systems in which the virus is replicating. In addition, the novel oligomers described herein enable further characterization of the HCV genome. Polynucleotide probes and primers derived from these sequences may be used to amplify sequences present in cDNA libraries, and/or to screen cDNA libraries for additional overlapping cDNA sequences, which, in turn, may be used to obtain more overlapping sequences. As indicated in PCT Pub. No. WO90/14436, the genome of HCV appears to be RNA comprising primarily a large open reading frame (ORF) which encodes a large polyprotein.

In addition to the above, the information provided infra allows the identification of additional HCV strains or isolates. The isolation and characterization of the additional HCV strains or isolates may be accomplished by, for example, isolating the nucleic acids from body components which contain viral particles and/or viral RNA, creating cDNA libraries using oligomers based on the HCV1 sequence, for screening the libraries for clones containing HCV cDNA sequences described infra, and comparing the HCV cDNAs from the new isolates with the cDNAs described in PCT Pub. No. WO90/14436 and infra. Strains or isolates which fit within the parameters of HCV, as described in the Definitions section, supra, are readily identifiable. Other methods for identifying HCV strains will be obvious to those of skill in the art, based upon the information provided herein.

### Isolation of the HCV cDNA Sequences

The oligomers of the invention contain regions which form hybrid duplex structures with targeted sequences in HCV polynucleotides. The HCV polynucleotide hybridizing regions of the oligomers may be ascertained from the HCV cDNA sequence(s) provided herein, and described in PCT Pub. No. WO90/14436. A composite of HCV cDNA from HCV1, a prototypic HCV, is shown in Fig. 1. The composite sequence is based upon sequence information derived from a number of HCV cDNA clones, which were isolated from a number of HCV cDNA libraries, including the "c" library present in lambda gtll (λgt11) (ATCC No. 40394), and from human serum. The HCV cDNA clones were isolated by methods described in PCT Pub. No. WO90/14436. Briefly, the majority of clones which were isolated contained sequences from the HCV cDNA "c" library which was constructed using pooled serum from a chimpanzee with chronic HCV infection and containing a high titer of the virus, i.e., at least 10⁶ chimp infectious doses/ml (CID/ml). The pooled serum was used to isolate viral particles; nucleic acids isolated from these particles were used as the template in the construction of cDNA libraries to the viral genome. The initial clone, 5-1-1, was obtained by screening the "c" library with serum from infected individuals. After the isolation of the initial clone, the remainder of the sequence was obtained by screening with synthetic polynucleotide probes, the sequences of which were derived from the 5'-region and the 3'-region of the known HCV cDNA sequence(s).

The description of the methods to retrieve the cDNA sequences is mostly of historical interest. The resultant sequences (and their complements) are provided herein, and the sequences, or any portion thereof, could be prepared using synthetic methods, or by a combination of synthetic methods with retrieval of partial sequences using methods similar to those described in PCT Pub. No. WO90/14436.

### Oligomer Probes and Primers

Using as a basis conserved regions of the HCV genome (as illustrated in Fig. 1), oligomers complementary to the parts of the HCV genome listed in claim 1 of 12 nucleotides or more can be prepared which hybridize with the positive strand(s) of HCV RNA or its complement, as well as to HCV cDNAs. These oligomers can serve as probes for the detection (including isolation and/or labeling) of polynucleotides which contain HCV nucleotide sequences, and/or as primers for the transcription and/or replication of targeted HCV sequences. The oligomers contain a targeting polynucleotide sequence, which is comprised of nucleotides which are complementary to a target HCV nucleotide sequence; the sequence is of sufficient length and complementarity with the HCV sequence to form a duplex which has sufficient stability for the purpose intended. For example, if the purpose is the isolation, via immobilization, of an analyte containing a target HCV sequence, the oligomers would contain a polynucleotide region which is of sufficient length and complementarity to the targeted HCV sequence to afford sufficient duplex stability to immobilize the analyte on a solid surface, via its binding to the oligomers, under the isolation conditions. For example, also, if the oligomers are to serve as primers for the transcription and/or replication of target HCV sequences in an analyte polynucleotide, the oligomers would contain a polynucleotide region of sufficient length and complementarity to the targeted HCV sequence to allow the polymerizing agent to continue replication from the primers which are in stable duplex form with the target sequence, under the polymerizing conditions. For example, also, if the oligomers are to be used as label probes, or are to bind to multimers, the targeting polynucleotide region would be of sufficient length and complementarity to form stable hybrid duplex structures with the label probes and/or multimers to allow detection of the duplex The oligomers may contain a minimum of 12 contiguous nucleotides which are complementary to the targeted HCV sequence and preferably will contain a minimum of about 14 contiguous nucleotides which are complementary to the targeted HCV sequence.

Suitable HCV nucleotide targeting sequences which are listed in claim 1 may be comprised of nucleotides which are complementary nucleotides selected from HCV cDNA nucleotides, which are shown in Fig. 1.

The oligomer, however, need not consist only of the sequence which is complementary to the targeted HCV sequence. It may contain in addition, nucleotide sequences or other moieties which are suitable for the purposes for which the oligomers are used. For example, if the oligomers are used as primers for the amplification of HCV sequences via PCR, they may contain sequences which, when in duplex, form restriction enzyme sites which facilitate the cloning of the amplified sequences. For example, also, if the oligomers are to be used as "capture probes" in hybridization assays (described infra), they would contain in addition a binding partner which is coupled to the oligomer containing the nucleotide sequence which is complementary to the targeted HCV sequence. Other types of moieties or sequences which are useful of which the oligomers may be comprised or coupled to, are those which are known in the art to be suitable for a variety of purposes, including the labeling of nucleotide probes.

The preparation of the oligomers is by means known in the art, including, for example, by methods which include excision, transcription, or chemical synthesis.

In the basic nucleic acid hybridization assay, single-stranded analyte nucleic acid (either DNA or RNA) is hybridized to a nucleic acid probe, and resulting duplexes are detected. The probes for HCV polynucleotides (natural or derived) are of a length which allows the detection of unique viral sequences by hybridization. Sequences of a minimum of 12 nucleotides are preferred, and about 20 nucleotides or more appears optimal. Preferably, these sequences will derive from regions which lack heterogeneity. These probes can be prepared using routine methods, including automated oligonucleotide synthetic methods. Among useful probes, for example, are those derived from the newly isolated clones disclosed herein, as well as the various oligomers useful in probing cDNA libraries, set forth below. A complement to any unique portion of the HCV genome will be satisfactory. For use as probes, complete complementarity is desirable, though it may be unnecessary as the length of the fragment is increased.

For use of such probes as agents to detect the presence of HCV polynucleotides (for example in screening for contaminated blood), the biological sample to be analyzed, such as blood or serum, may be treated, if desired, to extract the nucleic acids contained therein. The resulting nucleic acid from the sample may be subjected to gel electrophoresis or other size separation techniques; alternatively, the nucleic acid sample may be dot blotted without size separation. In order to form hybrid duplexes with the targeting sequence of the probe, the targeted region of the analyte nucleic acid must be in single stranded form. Where the sequence is naturally present in single stranded form, denaturation will not be required. However, where the sequence is present in double stranded form, the sequence will be denatured. Denaturation can be carried out by various techniques known in the art. Subsequent to denaturation, the analyte nucleic acid and probe are incubated under conditions which promote stable hybrid formation of the target sequence in the probe with the putative targeted sequence in the analyte, and the resulting duplexes containing the probe(s) are detected.

Detection of the resulting duplex, if any, is usually accomplished by the use of labeled probes; alternatively, the probe may be unlabeled, but may be detectable by specific binding with a ligand which is labeled, either directly or indirectly. Suitable labels, and methods for labeling probes and ligands are known in the art, and include, for example, radioactive labels which may be incorporated by known methods (e.g., nick translation or kinasing), biotin, fluorescent groups, chemiluminescent groups (e.g., dioxetanes, particularly triggered dioxetanes), enzymes, antibodies, and the like.

The region of the probes which are used to bind to the analyte can be made completely complementary to the HCV genome. Therefore, usually high stringency conditions are desirable in order to prevent false positives. However, conditions of high stringency should only be used if the probes are complementary to regions of the viral genome which lack heterogeneity. The stringency of hybridization is determined by a number of factors during hybridization and during the washing procedure, including temperature, ionic strength, length of time, and concentration of formamide. These factors are outlined in, for example, T. Maniatis (1982).

Variations of this basic scheme which are known in the art, including those which facilitate separation of the duplexes to be detected from extraneous materials and/or which amplify the signal from the labeled moiety, may also be used. A number of these variations are reviewed in, for example: Matthews and Kricka (1988), Anal. Biochem. 169:1; Landegren et al. (1988), Science 242:229; and Mittlin (1989), Clin. Chem. 35:1819. Probes suitable for detecting HCV in these assays are comprised of sequences which hybridize with target HCV polynucleotide sequences to form duplexes with the analyte strand, wherein the duplexes are of sufficient stability for detection in the specified assay system.

A suitable variation is, for example, one which is described in U.S. Patent No. 4,868,105, issued Sept. 9, 1989, and in E.P.O. Pub. No. 225807 (published June 16, 1987). These publications describe a solution phase nucleic acid hybridization assay in which the analyte nucleic acid is hybridized to a labeling probe set and to a capturing probe set. The probe-analyte complex is coupled by hybridization with a solid-supported capture probe that is complementary to the capture probe set. This permits the analyte nucleic acid to be removed from solution as a solid phase complex. Having the analyte in the form of a solid phase complex facilitates subsequent separation steps in the assay. The labeling probe set is complementary to a labeled probe that is bound through hybridization to the solid phase/analyte complex.

Generally, it is expected that the HCV genome sequences will be present in serum of infected individuals at relatively low levels, i.e., at approximately 10²-10³ chimp infectious doses (CID) per ml. This level may require that amplification techniques be used in hvbridization assays. Such techniques are known in the art. For example, the Enzo Biochemical Corporation "Bio-Bridge" (TM) system uses terminal deoxynucleotide transferase to add unmodified 3'-poly-dT-tails to a DNA probe. The poly dT-tailed probe is hybridized to the target nucleotide sequence, and then to a biotin-modified poly-A. PCT Pub. No. WO84/03520 and EPO Pub. No. 124221 describe a DNA hybridization assay in which: (1) analyte is annealed to a single-stranded DNA probe that is complementary to an enzyme-labeled oligonucleotide; and (2) the resulting tailed duplex is hybridized to an enzyme-labeled oligonucleotide. EPO Pub. No. 204510 describes a DNA hybridization assay in which analyte DNA is contacted with a probe that has a tail, such as a poly-dT tail, an amplifier strand that has a sequence that hybridizes to the tail of the probe, such as a poly-A sequence, and which is capable of binding a plurality of labeled strands. A type of hybridization assay which is described in EPO Pub. No. 317077 (published May 24, 1989), which should detect sequences at the level of approximately 10⁶/ml, utilizes nucleic acid multimers which bind to single-stranded analyte nucleic acid, and which also bind to a multiplicity of single-stranded labeled oligonucleotides. A particularly desirable technique may involve amplification of the target HCV sequences in sera approximately 10,000 fold (i.e., to approximately 10⁶ sequences/ml), as part of the hybridization system. The amplification may be accomplished, for example, by the polymerase chain reactions (PCR) technique described by Saiki et al. (1986), by Mullis, U.S. Patent No. 4,683,195, and by Mullis et al. U.S. Patent No. 4,683,202. Amplification may be prior to, or preferably subsequent to purification of the HCV target sequence. For example, amplification may be utilized in conjunction with the assay methods described in U.S. Patent No. 4,868,105, or if even further amplification is desired, in conjunction with the hybridization system described in EPO Pub. No. 317077.

Preferred methods for detecting HCV sequences in an analyte polynucleotide strand are based upon the hybridization detection methods described in U.S. Patent No. 4,868,105 and in EPO Pub. No. 317077. These methods are solution-phase sandwich hybridization assays which utilize both capture and label probes which hybridize to target sequences in an analyte nucleic acid. In the use of these assays to screen biological samples for HCV, the probes used would bind to conserved regions of the HCV genome. The capture and label probes may be interspersed in their binding to the target sequence. Alternatively, in a preferred mode the capture and label probes are in sets, and the probes of one set do not intersperse with the probes of another set. In the latter mode, preferably the set(s) of multiple capture probes hybridize to the most conserved regions of the genome, while the set(s) of multiple label probes may hybridize to regions which exhibit small amounts of divergence. For example, using the prototype HCV1 cDNA sequence shown in Fig. 1, probes could be used which hybridize to sequences in the region of nucleotides from about -318 to about 174, and/or nucleotides in the region of about 4378 to about 4902, and/or nucleotides in the region of from about 4056 to about 4448. The preferred probes would hybridize to sequences in the 5'-region of the HCV genome, since, as shown infra, this region appears to be highly conserved. Thus, preferred probes may hybridize to, for example, nucleotides from about -318 to about 174 as shown in Fig. 1. Probes could be used which hybridize to either the positive strand in conserved regions, and/or its complement, depending upon the purpose, for example, to detect viral genomic sequences, or to detect HCV cDNA sequences resulting from PCR amplification, or to detect replicative intermediates to the positive HCV RNA strand.

### Detection of HCV RNA and Polynucleotides Derived Therefrom Using an HCV/cPCR Method

A particularly useful method for detecting HCV RNA or polynucleotides derived from HCV RNA is the HCV/cPCR method, which is a subject of the herein application, and which utilizes the polymerase chain reaction technique (PCR) which is described by Saiki et al. (1986), by Mullis in U.S. Pat. No. 4,683,195, and by Mullis et al. in U.S. Patent No. 4,683,202. The HCV/cPCR method utilizes primers and probes derived from the information provided herein concerning the nature of the HCV genome.

Generally, in the PCR technique, short oligonucleotide primers are prepared which match opposite ends of a desired sequence. The sequence between the primers need not be known. A sample of polynucleotide is extracted and denatured, preferably by heat, and hybridized with oligonucleotide primers which are present in molar excess. Polymerization is catalyzed by a template- and primer-dependent polymerase in the presence of deoxynucleotide triphosphates or nucleotide analogs (dNTPs). This results in two "long products" which contain the respective primers at their 5'-termini, covalently linked to the newly synthesized complements of the original strands. The replicated DNA is again denatured, hybridized with oligonucleotide primers, returned to polymerizing conditions, and a second cycle of replication is initiated. The second cycle provides the two original strands, the two long products from cycle 1, and two "short products" replicated from the long products. The short products contain sequences (sense or antisense) derived from the target sequence, flanked at the 5'- and 3'-termini with primer sequences. On each additional cycle, the number of short products is replicated exponentially. Thus, this process causes the amplification of a specific target sequence.

In the method, a sample is provided which is suspected of containing HCV RNA, or a fragment thereof. The sample is usually taken from an individual suspected of having NANBH; however, other sources of the sample are included, e.g., conditioned medium or cells from in vitro systems in which the virus has been replicated. The sample, however, must contain the target nucleic acid sequence(s).

The sample is then subjected to conditions which allow reverse transcription of HCV RNA into HCV cDNA. Conditions for reverse transcribing RNA are known to those of skill in the art, and are described in, for example, Maniatis et al. (1982), and in Methods in Enzymology. A preferred method of reverse transcription utilizes reverse transcriptase from a variety of sources, including recombinant molecules, and isolated from, for example, a retrovirus, preferably from avian myeloblastosis virus (AMV), and suitable conditions for the transcription. The HCV cDNA product of reverse transcription is in a RNA:DNA hybrid, which results from the first round of reverse transcription; subsequently, DNA:DNA hybrids result from two or more rounds of transcription.

The HCV cDNA resulting from reverse transcription is then subjected to PCR to amplify the target sequence. In order to accomplish this, the HCV cDNA is denatured, and the separated strands are hybridized with primers which flank the target sequence.

Strand separation may be accomplished by any suitable denaturing method, including physical, chemical, or enzymatic means, which are known to those of skill in the art. A preferred method, which is physical, involves heating the nucleic acid until it is completely (> 99%) denatured. Typical heat denaturation involves temperatures ranging from about 80°C to about 105°C, for times ranging from about 1 to 10 minutes.

After hybridization of the HCV cDNA with the primers, the target HCV sequences are replicated by a polymerizing means which utilizes a primer oligonucleotide to initiate the synthesis of the replicate chain. The primers are selected so that they are complementary to sequences of the HCV genome. Oligomeric primers which are complementary to regions of the sense and antisense strands of HCV cDNA can be designed from the HCV cDNA sequences from the composite cDNA sequence provided in Fig. 1.

The primers are selected so that their relative positions along a duplex sequence are such that an extension product synthesized from one primer, when it is separated from its template (complement), serves as a template for the extension of the other primer to yield a replicate chain of defined length.

The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the agent for polymerization. The exact lengths of the primers will depend on many factors, including temperature and source of the primer and use of the method. For example, depending on the complexity of the target sequence, the oligonucleotide primer typically contains about 15-45 nucleotides, although it may contain more or fewer nucleotides. Short primer molecules generally require cooler temperatures to form sufficiently stable hybrid complexes with the template.

The primers used herein are selected to be "substantially" complementary to the different strands of each specific sequence to be amplified. Therefore, the primers need not reflect the exact sequence of the template, but must be sufficiently complementary to selectively hybridize with their respective strands. For example, a non-complementary nucleotide fragment may be attached to the 5'-end of the primer, with the remainder of the primer sequence being complementary to the strand. Alternatively, non-complementary bases or longer sequences can be interspersed into the primer, provided that the primer has sufficient complementarity with the sequence of one of the strands to be amplified to hybridize therewith, and to thereby form a duplex structure which can be extended by the polymerizing means. The non-complementary nucleotide sequences of the primers may include restriction enzyme sites. Appending a restriction enzyme site to the end(s) of the target sequence would be particularly helpful for cloning of the target sequence.

It will be understood that "primer", as used herein, may refer to more than one primer, particularly in the case where there is some ambiguity in the information regarding the terminal sequence(s) of the target region to be amplified. Hence, a "primer" includes a collection of primer oligonucleotides containing sequences representing the possible variations in the sequence or includes nucleotides which allow a typical basepairing. One of the primer oligonucleotides in this collection will be homologous with the end of the target sequence. A specific case is where oligomer sets are utilized to prime the amplification of a potentially variant region of the HCV genome.

It is anticipated that there will be a variety of strains or isolates of HCV with sequences which deviate from HCV1, the prototype strain. Therefore, in order to detect variant strains it is preferable to construct primers which hybridize to conserved regions of the HCV genome. The conserved regions may be determined by comparing the nucleotide or amino acid sequences of several HCV strains/isolates. There appear to be at least three regions of conserved amino acid in the HCV genome, described supra., from which primers may be derived. The primers described infra, in the Examples, are derived from what are believed to be conserved regions of HCV, based upon sequence homology to that of the Flaviviruses.

The oligonucleotide primers may be prepared by any suitable method. Methods for preparing oligonucleotides of specific sequence are known in the art, and include, for example, cloning and restriction of appropriate sequences, and direct chemical synthesis. Chemical synthesis methods may include, for example, the phosphotriester method described by Narang et al. (1979), the phosphodiester method disclosed by Brown et al. (1979), the diethylphosphoramidate method disclosed in Beaucage et al. (1981), and the solid support method in U.S. Patent No. 4.458,066.

The primers may be labeled, if desired, by incorporating means detectable by spectroscopic, photochemical, biochemical, immunochemical, or chemical means.

Template-dependent extension of the oligonucleotide primer(s) is catalyzed by a polymerizing agent in the presence of adequate amounts of the four deoxyribonucleotide triphosphates (dATP, dGTP, dCTP and dTTP) or analogs, in a reaction medium which is comprised of the appropriate salts, metal cations, and pH buffering system. Suitable polymerizing agents are enzymes known to catalyze primer- and template-dependent DNA synthesis. Known DNA polymerases include, for example, E. coli DNA polymerase I or its Klenow fragment, T₄ DNA polymerase, and Taq DNA polymerase. The reaction conditions for catalyzing DNA synthesis with these DNA polymerases are known in the art.

The products of the synthesis are duplex molecules consisting of the template strands and the primer extension strands, which include the target sequence. These products, in turn, serve as template for another round of replication. In the second round of replication, the primer extension strand of the first cycle is annealed with its complementary primer; synthesis yields a "short" product which is bounded on both the 5'- and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

The PCR method can be performed in a number of temporal sequences. For example, it can be performed step-wise, where after each step new reagents are added, or in a fashion where all of the reagents are added simultaneously, or in a partial step-wise fashion, where fresh reagents are added after a given number of steps.

In a preferred method, the PCR reaction is carried out as an automated process Which utilizes a thermostable enzyme. In this process the reaction mixture is cycled through a denaturing region, a primer annealing region, and a reaction region. A machine may be employed which is specifically adapted for use with a thermostable enzyme, which utilizes temperature cycling without a liquid handling system, since the enzyme need not be added at every cycle. This type of machine is commercially available from Perkin Elmer Cetus Corp.

After amplification by PCR, the target polynucleotides are detected by hybridization with a probe polynucleotide which forms a stable hybrid with that of the target sequence under stringent to moderately stringent hybridization and wash conditions. If it is expected that the probes will be completely complementary (i.e., about 99% or greater) to the target sequence, stringent conditions will be used. If some mismatching is expected, for example if variant strains are expected with the result that the probe will not be completely complementary, the stringency of hybridizat ?n may be lessened. However, conditions are chosen which rule out nonspecific/adventitious binding. Conditions which affect hybridization, and which select against nonspecific binding are known in the art, and are described in, for example, Maniatis et al. (1982). Generally, lower salt concentration and higher temperature increase the stringency of binding. For example, it is usually considered that stringent conditions are incubation in solutions which contain approximately 0.1 × SSC, 0.1% SDS, at about 65°C incubation/wash temperature, and moderately stringent conditions are incubation in solutions which contain approximately 1-2 × SSC, 0.1% SDS and about 50°-65°C incubation/wash temperature. Low stringency conditions are 2 X SSC and about 30°-50°C.

Probes for HCV target sequences may be derived from the HCV cDNA sequence shown in Fig. 1, or from new HCV isolates. The HCV probes may be of any suitable length which span the target region, but which exclude the primers, and which allow specific hybridization to the target region. If there is to be complete complementarity, i.e., if the strain contains a sequence identical to that of the probe, since the duplex will be relatively stable under even stringent conditions, the probes may be short, i.e., in the range of about 12-30 base pairs. If some degree of mismatch is expected with the probe, i.e., if it is suspected that the probe will hybridize to a variant region, the probe may be of greater length, since length seems to counterbalance some of the effect of the mismatch(es).

The probe nucleic acid having a sequence complementary to the target sequence may be synthesized using similar techniques described supra. for the synthesis of primer sequences. If desired, the probe may be labeled. Appropriate labels are described supra.

In some cases, it may be desirable to determine the length of the PCR product detected by the probe. This may be particularly true if it is suspected that variant HCV strains may contain deletions within the target region, or if one wishes to confirm the length of the PCR product. In such cases it is preferable to subject the products to size analysis as well as hybridization with the probe. Methods for determining the size of nucleic acids are known in the art, and include, for example, gel electrophoresis, sedimentation in gradients, and gel exclusion chromatography.

The presence of the target sequence in a biological sample is detected by determining whether a hybrid has been formed between the HCV polynucleotide probe and the nucleic acid subjected to the PCR amplification technique. Methods to detect hybrids formed between a probe and a nucleic acid sequence are known in the art. For example, for convenience, an unlabeled sample may be transferred to a solid matrix to which it binds, and the bound sample subjected to conditions which allow specific hybridization with a labeled probe; the solid matrix is than examined for the presence of the labeled probe. Alternatively, if the sample is labeled, the unlabeled probe is bound to the matrix, and after the exposure to the appropriate hybridization conditions, the matrix is examined for the presence of label. Other suitable hybridization assays are described supra.

### Determination of Variant HCV Sequences Using PCR

In order to identify variant HCV strains, and thereby to design probes for those variants, the above described HCV/cPCR method is utilized to amplify variant regions of the HCV genome, so that the nucleotide sequences of these variant target regions can be determined. Generally, variant types of HCV might be expected to occur in different geographic locations than that in which the HCV1 strain is predominant, for example, Japan, Africa, etc.; or in different vertebrate species which are also infected with the virus. Variant HCV may also arise during passage in tissue culture systems, or be the result of spontaneous or induced mutations.

In order to amplify the variant target region, primers are designed to flank the suspect region, and preferably are complementary to conserved regions. Primers to two regions of HCV which are probably conserved, based upon the Flavivirus model. These primers and probes may be designed utilizing the sequence information for the HCV1 strain provided in Fig. 1.

Analysis of the nucleotide sequence of the target region(s) may be by direct analysis of the PCR amplified products. A process for direct sequence analysis of PCR amplified products is described in Saiki et al. (1988).

Alternatively, the amplified target sequence(s) may be cloned prior to sequence analysis. A method for the direct cloning and sequence analysis of enzymatically amplified genomic segments has been described by Scharf (1986). In the method, the primers used in the PCR technique are modified near their 5'-ends to produce convenient restriction sites for cloning directly into, for example, an M13 sequencing vector. After amplification, the PCR products are cleaved with the appropriate restriction enzymes. The restriction fragments are ligated into the M13 vector, and transformed into, for example, a JM 103 host, plated out, and the resulting plaques are screened by hybridization with a labeled oligonucleotide probe. Other methods for cloning and sequence analysis are known in the art.

### Universal Primers for Flaviviruses and for HCV

Studies of the nature of the genome of the HCV, utilizing probes derived from the HCV cDNA, as well as sequence information contained within the HCV cDNA, are suggestive that HCV is a Flavi-like virus. These studies are described in PCT Pub. No. WO90/14436. A comparison of the HCV cDNA sequence derived from the HCV cDNA clones with known sequences of a number of Flaviviruses show that HCV contains sequences which are homologous to conserved sequences in the Flaviviruses. These conserved sequences may allow the creation of primers which may be universal in their application for amplification of target regions of Flaviviruses, and for HCV. Identification of the species is then accomplished utilizing a probe specific for the species. The genomes of a number of Flaviviruses are known in the art, and include, for example, Japanese Encephalitis Virus (Sumiyoshi et al. (1987)), Yellow Fever Virus (Rice et al. (1985)), Dengue Type 2 Virus (Hahn et al. (1988)), Dengue Type 4 Virus (Mackow (1987)), and West Nile Virus (Castle et al. (1986)). Identification of HCV RNA is accomplished utilizing a probe specific for HCV, the sequence of which can be determined the HCV cDNA sequences provided herein.

Alternatively, utilization of sets of probe(s) designed to account for codon degeneracy and therefore contain common sequences to the Flaviviruses and to HCV, as determined by a comparison of HCV amino acid sequences with the known sequences of the Flaviviruses, allows a general detection system for these viruses.

### Construction of Desired DNA Sequences

Synthetic oligonucleotides may be prepared using an automated oligonucleotide synthesizer as described by Warner (1984). If desired the synthetic strands may be labeled with ³²P by treatment with polynucleotide kinase in the presence of ³²P-ATP, using standard conditions for the reaction.

DNA sequences, including those isolated from cDNA libraries, may be modified by known techniques, including, for example site directed mutagenesis, as described by Zoller (1982). Briefly, the DNA to be modified is packaged into phage as a single stranded sequence, and converted to a double stranded DNA with DNA polymerase using, as a primer, a synthetic oligonucleotide complementary to the portion of the DNA to be modified, and having the desired modification included in its own sequence. The resulting double stranded DNA is transformed into a phage supporting host bacterium. Cultures of the transformed bacteria, which contain replications of each strand of the phage, are plated in agar to obtain plaques. Theoretically, 50% of the new plaques contain phage having the mutated sequence, and the remaining 50% have the original sequence. Replicates of the plaques are hybridized to labeled synthetic probe at temperatures and conditions which permit hybridization with the correct strand, but not with the unmodified sequence. The sequences which have been identified by hybridization are recovered and cloned.

### Kits for Screening for HCV Derived Polynucleotides

Oligomers which are probes and/or primers for amplification and/or screening of samples for HCV can be packaged into kits. Kits for screening for HCV sequences include the oligomeric probe DNAs. Kits for amplification of HCV sequences may include the oligomeric primers used in the amplification. The kits usually contain the probes or primers in a premeasured or predetermined amount, as well as other suitably packaged reagents and materials, in separate suitable containers, needed for the particular hybridization and/or amplification protocol(s). For example, the kit may contain standards, buffers, supports, enzymes, substrates, label probes, binding partners, and/or instructions for conducting the test.

### Examples

Described below are 2 examples (Examples I and II) of detection of HCV RNA using probes and primers not falling within the scope of the present claims and 1 Example (III) of the use of the oligonucleotides of claim 1 for the detection of HCV.

### I. Detection of Positive and Negative Strand 5'-HCV RNA in Serum

The RNA in HCV27. isolated from serum, was analyzed for the presence of positive and negative strands using the PCR method. The PCR method was performed essentially as described above, except for the following.

The extracted HCV27 RNA was reverse transcribed into single-stranded cDNA using as a primer either Alex90 or JH52. Alex90, which is derived from nucleotides -312 to -283 of the HCV1 genome, and has the sequence: 5' ACC ATG AAT CAC TCC CCT GTG AGG AAC TAC 3'. JH52 is derived from HCV nucleotides -93 to -117; the nucleotide numbers are indicated in parentheses below the sequences. In JH52 the underlined dinucleotide has been mutated to create the NotI site. The sequence pf JH52 is as follows: 
(Primer)    Stuffer   NotI         HCV sequence
(JH52)   5'  AGTCTT  GCGGCCGC       ACGCCCAAATC       3'
         (-93)                      (-117)
 The sequence of Alex90 matches that in nucleotides -312 to -283 of the positive strand of HCV RNA, whereas JH52 matches that of nucleotides -117 to -93 of the negative strand. The resulting single-stranded HCV cDNAs were each separately amplified by PCR using Alex90 and JH52. Detection of the amplified products was accomplished by Southern blotting, using Alex89 as the probe. Alex89 matches nucleotide numbers -203 to -175 of HCV RNA. The sequence of Alex89 is: 5' CCA TAG TGG TCT GCG GAA CCG GTG AGT ACA 3'. The analysis indicated that, by this method, the signals of the amplified products of both RNA strands were of equal intensity. These results are suggestive that HCV RNA in the 5'-region may exist as double-stranded RNA.

### II. Detection of HCV RNA in Plasma Using HCV/cPCR,Using Primers and Probes Derived from the 5'-Region of HCV RNA

### Extraction of HCV RNA from plasma

Frozen plasma was thawed in a P3 hood. A 0.2 ml aliquot of digestion mix (100 millimolar (mM) Tris-HCl, 2 mM EDTA, 200 mM NaCl, 20 microgram/milliliter (µg/ml) MS2 RNA, 0.5% SDS, and 2 mg/ml Proteinase K, pH 8) was added to a 2 ml microfuge tube and prewarmed to 37°C. The plasma (0.2 ml) was then added, and the mixture allowed to incubate at 60°C for 1 hour. Next, phenol (0.4 ml) was added, and the mixture vortexed 3 × for 30 seconds, allowing 30 seconds between each vortexing. The mixture was then centrifuged for 5 minutes, and the aqueous phase recovered. The organic phase was back-extracted with 0.1 ml TES, and the pooled aqueous phases extracted 2× with 1 volume phenol/chloroform/IAA, then with 1 volume chloroform. To the extract was added 1 µl (2 µg) glycogen (Boehringer Mannheim Corp.) and 25 µl NaOAc (3 M, pH 5.4) and 1.25 ml cold EtOH, and the product frozen on dry ice and spun 10 min. The pellet was dissolved in 100 µl distilled water, and 5 µl NaOAc (pH 5.4) added with 250 µl cold EtOH. The solution was frozen and spun again, and the resulting pellet dissolved in 10 µl dH₂O.

### cDNA Synthesis

In order to synthesize cDNA from the extracted RNA, initially 5 µl of the dissolved RNA was incubated with 4 µl water and 1 µl (1 µg or 166 pmols of 18-mer) of RT primer. The incubation was at 70°C for 3 minutes, followed by chilling on ice. The sequence of the RT primer was: 5' CCC AAC ACT ACT CGG CTA 3'. After the initial incubation, the following were added to the incubation mixture: 10 µl of 5× first strand buffer (250 mM Tris HCl, pH 8.3, 375 mM KCl, 15 mM MgCl₂, 50 mM dithiothreitol); 2.5 µl deoxynucleoside triphosphates (10 mM each); 2.5 µl reverse transcriptase (MMLV from BRL, 200 units per µl), and distilled water to bring the volume to 50 µl. The mixture was incubated at 37°C for 1 hour, heated at 90°C for 3 minutes, and chilled on ice.

### PCR Amplification

PCR amplification of the HCV cDNA produced above was conducted using the control and test reagents listed below, in the Table. In the Table, "RNA" indicates a control sample in which the RNA was extracted from an individual which was uninfected with HCV1; this sample was carried through the steps of cDNA synthesis and PCR amplification. "cDNA" indicates a control sample which was carried through the steps of cDNA synthesis and PCR amplification; however, in this case the aliquot of RNA was replaced with water during cDNA synthesis. "Template" was a control for the PCR reaction from which the template was omitted. "Sample" indicates the serum which was tested for the presence of HCV RNA.

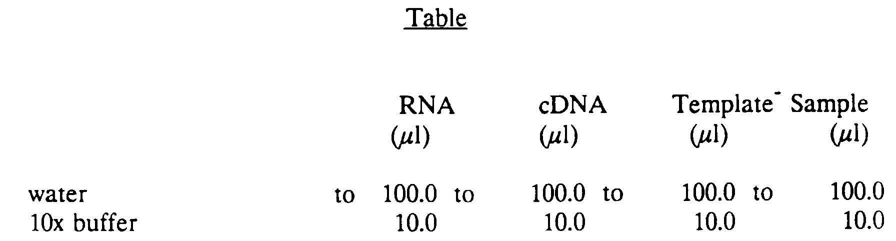

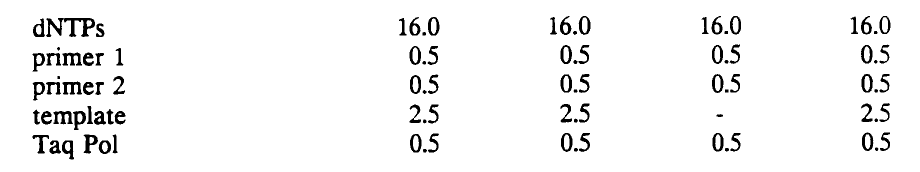

Primer 1 and Primer 2 were 0.5 µg/µl and 0.42 µg/µl, respectively, and had the following sequences. Primer 1:	5' ACC ATG AAT CAC TCC CCT GTG AGG AAC TAC 3' Primer 2: 5' AGT CTT GCG GGG GCA CGC CCA AAT C 3' These primers hybridize to a conserved region in the 5' end of the HCV genome. The samples contained 12.5 µl serum equivalents of HCV cDNA, The amplification cycle conditions used were as follows:
- 35 cycles:: Melting - 94°C for 1.5 min
Annealing - 60°C for 2 min
Elongation - 72°C for 3 min
- Final Elongation:: 72°C for 30 min
Soak at 4°C until removed.

The amplified product was probed using a labeled DNA. The sequence of the probe was the following:

Probe: 5' TTT CTT GGA TCA ACC CGC TCA ATG CCT
GGA 3'.

Over 200 HCV sera-positive samples were examined by the above procedure. Only results in which the controls were negative were considered. In this case, all sera-positive samples were also positive in the PCR assay.

### III. Probes Derived from the Putative "Core" Region of HCV RNA

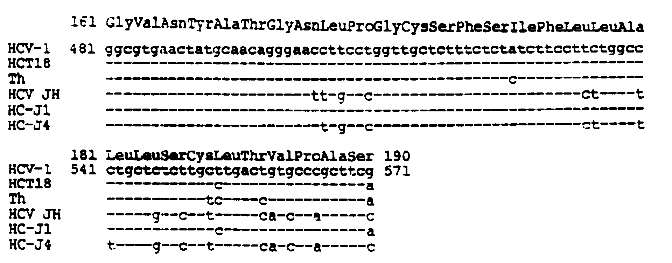

An analysis of the nucleotide sequences of cDNAs to different HCV isolates shows that a high degree of sequence conservation exists in the region from about nucleotide 1 to about nucleotide 570, using the numbering system for nucleotides shown in Fig. 1. This region putatively encodes a "core" polypeptide of HCV. The consensus sequences for five different isolates from different geographic locations (Japan and the U.S.) is shown in Fig. 2, where HCV1 is the prototype HCV; the amino acids encoded in the large ORF of HCV1 are shown above the consensus nucleotide sequences. In the sequences shown in Fig. 2, HCV-JH is from a personal communication from Dr. Tetsu Miyamura (National Institute of Health of Japan), and JC-J1 and JC-J4 are from Mayumi et al. (1990). It may be seen in Fig. 2 that between the consensus sequences in the putative "core" region, there is at least 90% homology relative to the sequence in HCV1. In view of the high degree of homology in the region between nucleotides +1 to +571, probes to this area would be useful in screening for HCV positive biological specimens.

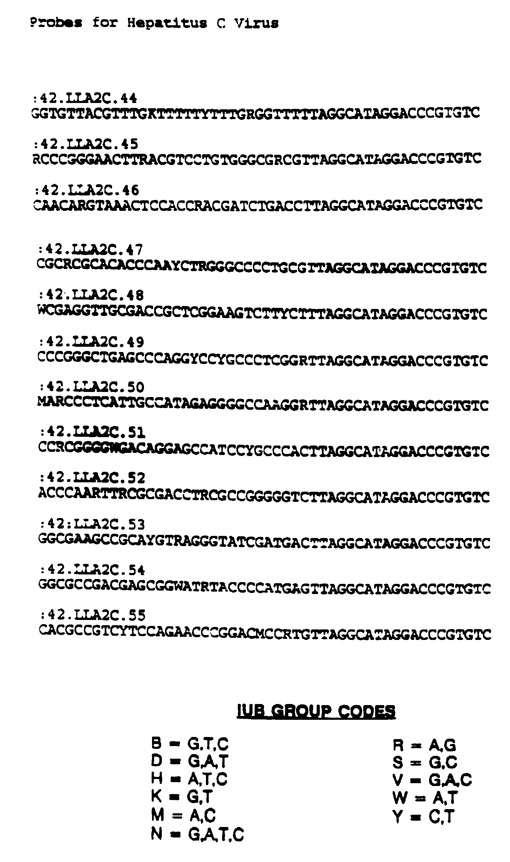

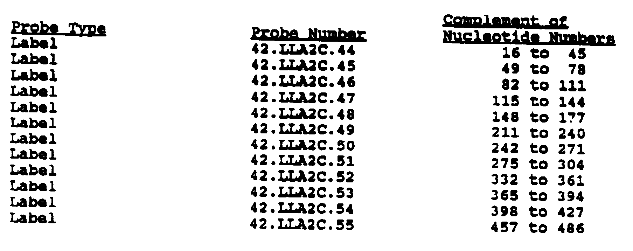

A set of label probes which may be used for the detection of HCV RNA from the region which putatively encodes an HCV "core" polypeptide are shown in Fig. 3. In Fig. 3, a "probe number" in the set includes a series of polynucleotides with heterogeneities indicated by the by the IUB Group Code listed in Fig. 3. The heterogeneities are to accomodate nucleotide sequence differences found in the consensus sequences of the different isolates. The regions of the HCV sequence to which the probes in the probe set of Fig. 3 are complementary are shown in Fig. 4, in which the nucleotide numbers correspond to the numbering in Fig. 1.

This probe set were used in an assay to detect HCV sequences. The assay format was described in PCT Pub. No. WO90/14436 in the Example section titled "Probes for Sandwich Hybidization for HCV."

### Industrial Applicability

The methods described herein, as well as the oligomers, both probes and primers, derived from HCV cDNA, and kits containing them, are useful for the accurate, relatively simple, and economic determination of the presence of HCV in biological samples, more particularly in blood which may be used for transfusions, and in individuals suspected of having HCV an infection. Moreover, these methods and oligomers may be useful for detecting an earlier stage of HCV infection than are immunological assays based upon the use of a recombinant HCV polypeptides. Also, an amplified polynucleotide hybridization assay detects HCV RNA in occasional samples which are anti-HCV antibody negative. Thus, the probes and primers described herein may be used amplified hybridization assays, in conjunction with an immunoassays based on HCV polypeptides to more completely identify infections due to HCV, and HCV-infected biological specimens, including blood.

The information provided herein allows the design of primers and/or probes which are derived from conserved regions of the HCV genome. The provision of these primers and probes makes available a general method which will detect variant HCV strains, and which will be of use in the screening of blood and blood products.

If the primers used in the method are derived from conserved regions of the HCV genome, the method should aid in the detection and/or identification of variant strains of HCV. This, in turn. should lead to the development of additional immunological reagents for the detection and diagnosis of HCV, as well as the development of additional polynucleotide reagents for detection and or treatment of HCV.

In addition, sets of primers and probes designed from the conserved amino acid sequences of Flaviviruses and HCV allow for a universal detection method for these infectious agents.

The following listed materials are on deposit under the terms of the Budapest Treaty with the American Type Culture Collection (ATCC), 12301 Parklawn Dr., Rockville, Maryland 20852, and have been assigned the following Accession Numbers.

| lambda-gt11 | ATCC No. | Deposit Date |
|---|---|---|
| HCV cDNA library | 40394 | 1 Dec. 1987 |
| clone 81 | 40388 | 17 Nov. 1987 |
| clone 91 | 40389 | 17 Nov. 1987 |
| clone 1-2 | 40390 | 17 Nov. 1987 |
| clone 5-1-1 | 40391 | 18 Nov. 1987 |
| clone 12f | 40514 | 10 Nov. 1988 |
| clone 35f | 40511 | 10 Nov. 1988 |
| clone 15e | 40513 | 10 Nov. 1988 |
| clone K9-1 | 40512 | 10 Nov. 1988 |
| JSC 308 | 20879 | 5 May 1988 |
| pS356 | 67683 | 29 April 1988 |

In addition, the following deposits were made on 11 May 1989.

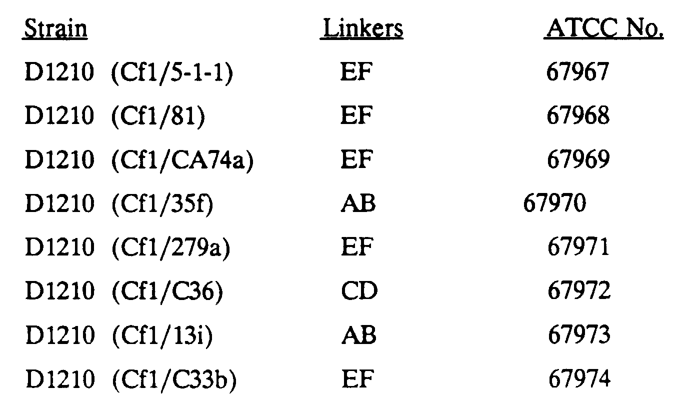

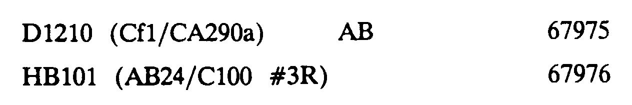

The following derivatives of strain D1210 were deposited on 3 May 1989.

| Strain Derivative | ATCC No. |
|---|---|
| pCF1CS/C8f | 67956 |
| pCF1AB/C12f | 67952 |
| pCF1EF/14c | 67949 |
| pCF1EF/15e | 67954 |
| pCF1AB/C25c | 67958 |
| pCF1EF/C33c | 67953 |
| pCF1EF/C33f | 67050 |
| pCF1CD/33g | 67951 |
| pCF1CD/C39c | 67955 |
| pCF1EF/C40b | 67957 |
| pCF1EF/CA167b | 67959 |

The following strains were deposited on May 12, 1989.

| Strain | ATCC No. |
|---|---|
| Lambda gt11(C35) | 40603 |
| Lambda gt10(beta-5a) | 40602 |
| D1210 (C40b) | 67980 |
| D1210 (M16) | 67981 |

The following biological materials were deposited on March 23, 1990.

| Material | ATCC No. |
|---|---|
| 5'-clone32 (in pUC18S) | 68276 |

## Claims

1. A process for detecting an HCV sequence in an analyte strand suspected of containing an HCV polynucleotide, wherein the HCV polynucleotide comprises a selected target region, said process comprising:
(a) providing an oligonucleotide capable of hybridizing to an HCV sequence in an analyte polynucleotide strand, wherein the oligonucleotide consists essentially of (i): a polynucleotide sequence selected from the group consisting of oligonucleotides at least 12 nucleotides in length complementary to the following regions of the HCV genome (as shown in Figure 1): 16-45, 49-78, 82-111, 115-144, 148-177, 211-240, 242-271, 275-304, 332-361, 365-394, 398427, and 457486, optionally linked to (ii) a polynucleotide other than that to which it is linked in nature;
(b) incubating the analyte strand with the oligonucleotide of (a) which allow specific hybrid duplexes to form between the targeting sequence and the target sequence; and
(c) detecting hybrids formed between target region, if any, and the oligonucleotide.

2. A process for detecting the presence or absence of a single or double-stranded specific nucleic acid sequence in a sample containing a nucleic acid or mixture thereof, which process comprises:
(a) treating the sample with one oligonucleotide primer for each strand of the specific nucleotide sequence, allowing specific hybrid duplexes to form between the oligonucleotide primer and the specific sequence;
(b) synthesizing a primer extension product; and
(c) detecting hybrids formed between the specific nucleotide sequence, if any, and the oligonucleotide primer;
characterized in that the oligonucleotide primers of (a) are as defined in claim 1 or the complement thereof.

3. The process of claim 2 wherein steps (a) and (b) are repeated.

4. The process of claim 2 wherein, said specific nucleic sequence is single stranded.

5. The process of claim 2 wherein, said nucleotide sequence is RNA and said primer is an oligodeoxyribonucleotide.

6. The process of any one of claims 1 to 5 wherein the oligonucleotide employed contains a restriction site on its 5' end.

7. The process according to any one of claims 1 to 6 when the oligonucleotide consists essentially of a polynucleotide sequence selected from the group consisting of oligonucleotides at least 12 nucleotides in length complementary to the following regions of the HCV genome (as shown in Figure 1): 16-45, 49-78, 82-111, 115-144, 148-177, 211-240, 242-271, 275-304, 332-361, 365-394, 398427, and 457-486.

8. A kit for detecting a specific nucleotide sequence, comprising an oligonucleotide primer capable of hybridizing to the specific nucleotide sequence in an analyte polynucleotide strand, characterized in that the specific nucleotide sequence to be detected is an HCV sequence and the oligomer consists essentilly of (i): a polynucleotide sequence selected from the group consisting of oligonucleotides at least 12 nucleotides in length complementary to the following regions of the HCV genome (as shown in Figure 1): 1645, 49-78, 82-111, 115-144, 148-177, 211-240, 242-271, 275-304, 332-361, 365-394, 398427, and 457486, optionally linked to (ii) a polynucleotide other than that to which it is linked in nature;

9. A method for eliminating blood contaminated with an infectious agent from a blood supply made up of units from individual blood donors comprising:
(a) providing analyte nucleic acids from a sample of blood suspected of containing a target sequence of a viral agent;
(b) providing an oligomer capable of hybridizing to the target sequence, if present, in the analyte nucleic acids, characterized in that
(i) the infectious agent is HCV; and
(ii) the oligomer consists essentilly of (i): a polynucleotide sequence selected from the group consisting of oligonucleotides at least 12 nucleotides in length complementary to the following regions of the HCV genome (as shown in Figure 1): 16-45, 49-78, 82-111, 115-144, 148-177, 211-240, 242-271, 275-304, 332-361, 365-394, 398427, and 457486, optionally linked to (ii) a polynucleotide other than that to which it is linked in nature;
(c) reacting (a) and (b) under conditions which allow the formation of a polynucleotide duplex between the targeting sequence and the target sequence, if any;
(d) detecting a duplex formed in (c), if any; and
(e) saving the blood from which complexes were not detected in (d).

10. A reagent useful for detecting of HCV, wherein said reagent comprises an oligonucleotide consisting essentially of polynucleotide sequence selected from the group consisting of oligonucleotides at least 12 nucleotides in length complementary to the following regions of the HCV genome (as shown in Figure 1): 16-45, 49-78, 82-111, 115-144, 148-177, 211-240, 242-271, 275-304, 332-361, 365-394, 398-427, and 457-486, optionally linked to (ii) a polynucleotide other than that to which it is linked in nature.

11. A reagent according to claim 10 which consists essentially of of a polynucleotide sequence selected from the group consisting of oligonucleotides at least 12 nucleotides in length complementary to the following regions of the HCV genome (as shown in Figure 1): 16-45, 49-78, 82-111, 115-144, 148-177, 211-240, 242-271, 275-304, 332-361, 365-394, 398-427, and 457-486.

12. The reagent of claim 10 or 11, wherein said oligonucleotide further comprises a detectable label.

## Patentansprüche

1. Verfahren zum Nachweis einer HCV-Sequenz in einem zu analysierenden Strang, bei dem vermutet wird, daß er ein HCV-Polynucleotid enthält, wobei das HCV-Polynucleotid eine ausgewählte Zielregion enthält, umfassend:
(a) Bereitstellung eines Oligonucleotides, das in der Lage ist, an eine HCV-Sequenz in einem zu analysierenden Polynucleotidstrang zu hybridisieren, wobei das Oligonucleotid im wesentlichen besteht aus (i): einer Polynucleotidsequenz, ausgewählt aus der Gruppe, bestehend aus Oligonucleotiden einer Länge von wenigstens 12 Nucleotiden, die komplementär zu den folgenden Abschnitten des HCV-Genomes (wie in Figur 1 gezeigt) sind: 16-45, 49-78, 82-111, 115-144, 148-177, 211-240, 242-271, 275-304, 332-361, 365-394, 398-427 und 457-486, gegebenenfalls gebunden an (ii) ein anderes Polynucleotid als das, an welches es natürlicherweise gebunden ist;
(b) Inkubation des zu analysierenden Stranges mit dem Oligonucleotid aus (a), die die Bildung von spezifischen Hybridduplexen zwischen der zielspezifischen Sequenz und der Zielsequenz ermöglicht, und
(c) Nachweis der Hybride, die aus einer gegebenenfalls vorhandenen Zielregion und dem Oligonucleotid gebildet wurden.

2. Verfahren zum Nachweis des Vorhandenseins oder der Abwesenheit einer einzel- oder doppelsträngigen spezifischen Nucleinsäuresequenz in einer Probe, die eine Nucleinsäure oder ein Gemisch davon enthält, umfassend:
(a) Behandlung der Probe mit einem Oligonucleotidprimer für jeden Strang der spezifischen Nucleotidsequenz, so daß die Bildung von spezifischen Hybridduplexen zwischen dem Oligonucleotidprimer und der spezifischen Sequenz möglich ist;
(b) Synthese eines Primerextensionsproduktes; und
(c) Nachweis von Hybriden, die zwischen der gegebenenfalls vorhandenen spezifischen Nucleotidsequenz und dem Oligonucleotidprimer entstanden sind;
dadurch **gekennzeichnet**, daß die Oligonucleotidprimer in (a) diejenigen sind, die in Anspruch 1 definiert wurden, oder deren komplementäre Stränge sind.

3. Verfahren nach Anspruch 2, dadurch **gekennzeichnet**, daß die Stufen (a) und (b) wiederholt werden.

4. Verfahren nach Anspruch 2, dadurch **gekennzeichnet**, daß die spezifische Nucleinsäuresequenz einzelsträngig ist.

5. Verfahren nach Anspruch 2, dadurch **gekennzeichnet**, daß die Nucleotidsequenz RNA ist und daß der Primer ein Oligodesoxyribonucleotid ist.

6. Verfahren nach einem der Ansprüche 1 bis 5, dadurch **gekennzeichnet**, daß das verwendete Oligonucleotid eine Restriktionsspaltstelle an seinem 5'-Ende enthält.

7. Verfahren nach einem der Ansprüche 1 bis 6, dadurch **gekennzeichnet**, daß das Oligonucleotid im wesentlichen aus einer Polynucleotidsequenz besteht, die ausgewählt ist aus der Gruppe, bestehend aus Oligonucleotiden einer Länge von wenigstens 12 Nucleotiden, komplementär zu den folgenden Regionen des HCV-Genomes (wie in Figur 1 gezeigt): 16-45, 49-78, 82-111, 115-144, 148-177, 211-240, 242-271, 275-304, 332-361, 365-394, 398-427 und 457-486.

8. Ein Kit zum Nachweis einer spezifischen Nucleotidsequenz, umfassend einen Oligonucleotidprimer, der in der Lage ist, an die spezifische Nucleotidsequenz in einem zu analysierenden Polynucleotidstrang zu hybridisieren, dadurch **gekennzeichnet**, daß die spezifische Nucleotidsequenz, die nachgewiesen werden soll, eine HCV-Sequenz ist und daß das Oligomer im wesentlichen besteht aus (i): einer Polynucleotidsequenz, ausgewählt aus der Gruppe, umfassend Oligonucleotide einer Länge von wenigstens 12 Nucleotiden, komplementär zu den folgenden Regionen des HCV-Genomes (wie in Figur 1 gezeigt): 16-45, 49-78, 82-111, 115-144, 148-177, 211-240, 242-271, 275-304, 332-361, 365-394, 398-427 und 457-486, gegebenenfalls gebunden an (ii) ein anderes Polynucleotid als das, an welches sie natürlicherweise gebunden ist.

9. Verfahren zur Entfernung von Blut, das mit einem infektiösen Agens kontaminiert ist, aus einer Blutbereitstellung, die sich aus Einheiten von einzelnen Blutspendern zusammensetzt, umfassend:
(a) Bereitstellung von zu analysierenden Nucleinsäuren aus einer Blutprobe, die im Verdacht steht, eine Zielsequenz eines viralen Agens zu enthalten;
(b) Bereitstellung eines Oligomers, das in der Lage ist, an die gegebenenfalls vorhandene Zielsequenz in den zu analysierenden Nucleinsäuren zu hybridisieren, dadurch **gekennzeichnet**, daß
(i) das infektiöse Agens HCV ist; und
(ii) das Oligomer im wesentlichen besteht aus (i): einer Polynucleotidsequenz, ausgewählt aus der Gruppe, umfassend Oligonucleotide einer Länge von wenigstens 12 Nucleotiden, komplementär zu den folgenden Regionen des HCV-Genomes (wie in Figur 1 gezeigt): 16-45, 49-78, 82-111, 115-144, 148-177, 211-240, 242-271, 275-304, 332-361, 365-394, 398-427 und 457-486, gegebenenfalls gebunden an (ii) ein anderes Polynucleotid als das, an welches sie natürlicherweise gebunden sind;
(c) Umsetzung von (a) und (b) unter Bedingungen, die die Bildung eines Polynucleotidduplexes zwischen der zielspezifischen Sequenz und der gegebenenfalls vorhandenen Zielsequenz erlaubt;
(d) Nachweis des in (c) gegebenenfalls gebildeten Duplexes; und
(e) Aufheben des Blutes, bei dem in (d) keine Komplexe nachgewiesen wurden.

10. Reagens, nützlich zum Nachweis von HCV, dadurch **gekennzeichnet**, daß das Reagens ein Oligonucleotid umfaßt, das im wesentlichen aus einer Polynucleotidsequenz besteht, die ausgewählt ist aus der Gruppe, umfassend Oligonucleotide einer Länge von wenigstens 12 Nucleotiden, komplementär zu den folgenden Regionen des HCV-Genomes (wie in Figur 1 gezeigt): 16-45, 49-78, 82-111, 115-144, 148-177, 211-240, 242-271, 275-304, 332-361, 365-394, 398-427 und 457-486, gegebenenfalls gebunden an (ii) ein anderes Polynucleotid als das, an welches es natürlicherweise gebunden ist.

11. Reagens nach Anspruch 10, das im wesentlichen aus einer Polynucleotidsequenz besteht, die ausgewählt ist aus der Gruppe, bestehend aus Oligonucleotiden einer Länge von wenigstens 12 Nucleotiden, komplementär zu den folgenden Regionen des HCV-Genomes (wie in Figur 1 gezeigt) : 16-45, 49-78, 82-111, 115-144, 148-177, 211-240, 242-271, 275-304, 332-361, 365-394, 398-427 und 457-486.

12. Reagens nach Anspruch 10 oder 11, dadurch **gekennzeichnet**, daß das Oligonucleotid zusätzlich eine nachweisbare Markierung enthält.

## Revendications

1. Procédé pour détecter une séquence d'HCV (virus de l'hépatite C) dans un brin analyte suspecté de contenir un polynucléotide d'HCV, dans lequel le polynucléotide d'HCV comprend une région cible choisie, ledit procédé comprenant les étapes consistant:
(a) à fournir un oligonucléotide capable de s'hybrider à une séquence d'HCV dans un brin polynucléotidique analyte, l'oligonucléotide consistant essentiellement en (i) : une séquence polynucléotidique choisie dans le groupe constitué par des oligonucléotides ayant une longueur d'au moins 12 nucléotides complémentaires des régions suivantes du génome d'HCV (tel que représenté à la figure 1): 16-45, 49-78, 82-111, 115-144, 148-177, 211-240, 242-271, 275-304, 332-361, 365-394, 398-427, et 457-486, éventuellement liée à (ii) un polynucléotide autre que celui auquel elle est liée à l'état naturel;
(b) à incuber le brin analyte avec l'oligonucléotide de (a) ce qui permet à des duplexes hybrides spécifiques de se former entre la séquence de ciblage et la séquence cible; et
(c) à détecter les hybrides formés entre la région cible, si elle présente, et l'oligonucléotide.

2. Procédé pour détecter la présence ou l'absence d'une séquence acide nucléique spécifique simple ou double brin dans un échantillon contenant un acide nucléique ou un mélange d'acides nucléiques, procédé qui comprend les étapes consistant:
(a) à traiter l'échantillon avec une amorce oligonucléotidique pour chaque brin de la séquence nucléotidique spécifique, permettre à des duplexes hybrides spécifiques de se former entre l'amorce oligonucléotidique et la séquence spécifique;
(b) à synthétiser un produit d'élongation de l'amorce; et
(c) à détecter les hybrides formés entre la séquence nucléotidique spécifique, si elle est présente, et l'amorce oligonucléotidique;
caractérisé par le fait que les amorces oligonucléotidiques de (a) sont celles définies à la revendication 1 ou leurs complémentaires.

3. Procédé selon la revendication 2 dans lequel les étapes (a) et (b) sont répétées.

4. Procédé selon la revendication 2 dans lequel, ladite séquence nucléique spécifique est simple brin.

5. Procédé selon la revendication 2 dans lequel, ladite séquence nucléotidique est de l'ARN et ladite amorce est un oligodésoxyribonucléotide.

6. Procédé selon l'une quelconque des revendications 1 à 5 dans lequel l'oligonucléotide employé contient un site de restriction sur son extrémité 5'.

7. Procédé selon l'une quelconque des revendications 1 à 6 dans lequel l'oligonucléotide consiste essentiellement en une séquence polynucléotidique choisie dans le groupe constitué par des oligonucléotides ayant une longueur d'au moins 12 nucléotides complémentaires des régions suivantes du génome d'HCV (comme présenté à la figure 1) : 16-45, 49-78, 82-111, 115-144, 148-177, 211-240, 242-271, 275-304, 332-361, 365-394, 398-427, et 457-486.

8. Kit pour la détection d'une séquence nucléotidique spécifique, comprenant une amorce oligonucléotidique capable de s'hybrider à la séquence nucléotidique spécifique dans un brin polynucléotidique analyte caractérisé par le fait que la séquence nucléotidique spécifique à détecter est une séquence d'HCV et l'oligomère consiste essentiellement en (i) une séquence polynucléotidique choisie dans le groupe constitué par des oligonucléotides ayant une longueur d'au moins 12 nucléotides complémentaires des régions suivantes du génome d'HCV (tel que présenté à la figure 1) : 16-45, 49-78, 82-111, 115-144, 148-177, 211-240, 242-271, 275-304, 332-361, 365-394, 398-427 et 457-486, éventuellement liée à (ii) un polynucléotide autre que celui auquel elle est liée à l'état naturel.

9. Méthode pour éliminer du sang contaminé par un agent infectieux d'une source de sang constituée d'unités provenant de donneurs de sang individuels comprenant les étapes consistant:
(a) à fournir des acides nucléiques analytes provenant d'un échantillon de sang suspecté de contenir une séquence cible d'un agent viral;
(b) à fournir un oligomère capable de s'hybrider à la séquence cible, si elle est présente, dans les acides nucléiques analytes, caractérisée par le fait que
(i) l'agent infectieux est HCV; et
(ii) l'oligomère consiste essentiellement en (i) : une séquence polynucléotidique choisie dans le groupe constitué par des oligonucléotides ayant une longueur d'au moins 12 nucléotides complémentaires des régions suivantes du génome d'HCV (tel que présenté à la figure 1) : 16-45, 49-78, 82-111, 115-144, 148-177, 211-240, 242-271, 275-304, 332-361, 365-394, 398427 et 457-486, éventuellement liée à (ii) un polynucléotide autre que celui auquel elle est liée à l'état naturel;
(c) à faire réagir (a) et (b) dans des conditions qui permettent la formation d'un duplexe polynucléotidique entre la séquence de ciblage et la séquence cible, si elle est présente;
(d) à détecter un duplexe formé en (c), si il est présent; et
(e) à garder le sang à partir duquel des complexes n'ont pas été détectés en (d).

10. Réactif utilisable pour la détection d'HCV, dans lequel ledit réactif comprend un oligonucléotide consistant essentiellement en une séquence polynucléotidique choisie dans le groupe constitué par des oligonucléotides ayant une longueur d'au moins 12 nucléotides complémentaires des régions suivantes du génome d'HCV (tel que présenté à la figure 1) : 16-45, 49-78, 82-111, 115-144, 148-177, 211-240, 242-271, 275-304, 332-361, 365-394, 398-427 et 457-486, éventuellement liée à (ii) un polynucléotide autre que celui auquel elle est liée à l'état naturel.

11. Réactif selon la revendication 10 qui consiste essentiellement en une séquence polynucléotidique choisie dans le groupe constitué par des oligonucléotides ayant une longueur d'au moins 12 nucléotides complémentaires des régions suivantes du génome d'HCV (tel que présenté à la figure 1) : 16-45, 49-78, 82-111, 115-144, 148-177, 211-240, 242-271, 275-304, 332-361, 365-394, 398-427 et 457-486.

12. Réactif selon la revendication 10 ou 11, dans lequel ledit oligonucléotide comprend en outre un marqueur détectable.
